# EUROPEAN PATENT SPECIFICATION

(11) **EP 2 294 383 B1**
(45) Date of publication and mention of the grant of the patent: **21.03.2018**
(21) Application number: 09749625.1
(22) Date of filing: 20.05.2009
(51) Int. Cl.: G01N 17/00, G01F 23/296, G01B 17/02, G01S 15/10, G01N 29/07, G01N 29/12, G01N 29/46, G01N 29/00, G01N 33/34

(54) **METHOD AND DEVICE FOR A HIGH PRECISION MEASUREMENT OF A CHARACTERISTIC OF A FOULING AND/OR SCALING DEPOSIT INSIDE A FLUID VESSEL OR OF A CHARACTERISTIC OF A PORTION OF THE WALL INSIDE A FLUID VESSEL BY USING AN ULTRASONIC TRANSDUCER**
VERFAHREN UND VORRICHTUNG ZUR HOCHGENAUEN MESSUNG EINER EIGENSCHAFT EINER VERSCHMULZUNG UND/ODER KALKABLAGERUNG IN EINEM FLÜSSIGKEITSBEHÄLTER ODER EINER EIGENSCHAFT EINES WANDTEILS IM INNEREN EINES FLÜSSIGKEITSBEHÄLTERS ANHAND EINES ULTRASCHALLKOPFES
PROCÉDÉ ET DISPOSITIF POUR UNE MESURE HAUTE PRÉCISION D'UNE CARACTÉRISTIQUE D'UN DÉPÔT DE SALISSURE ET/OU D'ENTARTRAGE À L'INTÉRIEUR D'UN RÉCIPIENT DE FLUIDE OU D'UNE CARACTÉRISTIQUE D'UNE PARTIE DE LA PAROI À L'INTÉRIEUR D'UN RÉCIPIENT DE FLUIDE À L'AIDE D'UN TRANSDUCTEUR ULTRASONIQUE

(30) Priority: 23.05.2008 EP 08009467
(43) Date of publication of application: 16.03.2011
(73) Proprietor: Solenis Technologies Cayman, L.P., 8200 Schaffhausen (CH)
(72) Inventor: SEIDA, Frank, 59368 Werne (DE); FLOCKEN, Christian, 47839 Krefeld (DE); PÖSCHMANN, Rainer, 47918 Tönisvorst (DE); SCHULTZ, Michael, Dr., D-06231 Bad Dürrenberg (DE); KLAUA, Robert, 06440 Halle (DE); DIETRICH, Georg, 06179 Zappendorf (DE)
(74) Representative: LKGLOBAL Lorenz & Kopf PartG mbB Patentanwälte
(86) International application number: PCT/EP2009/003597
(87) International publication number: WO 2009/141135

(56) References cited:
- WO-A-2007/093374
- DE-A1- 10 214 678
- US-A- 5 629 865
- US-A1- 2002 166 383
- US-A1- 2003 010 125
- Tarjei Rommetveit ET AL: "USING ULTRASOUND MEASUREMENTS FOR REAL-TIME PROCESS CONTROL OF PIPELINES AND PROCESS EQUIPMENT SUBJECTED TO CORROSION AND/OR EROSION", Proceedings of the 2008 NACE Corrosion Conference & Expo, 20 March 2008 (2008-03-20), pages 1-13, XP055361323,
- Daniel J Cotter ET AL: "High frequency ultrasonic thickness and acoustic velocity measurement methods for advanced material and component characterization", NDT.net, vol. 7, no. 10, 30 June 2002 (2002-06-30), pages 1-12, XP055361331,

## Description

### FIELD OF THE INVENTION

The invention relates to a method and a device for a high precision measurement of a characteristic of a fouling and/or scaling deposit inside a fluid vessel or of a characteristic of a portion of the wall inside a fluid vessel by using an ultrasonic transducer. The invention further relates to the use of such a device and to a method of monitoring a characteristic of a fouling and/or scaling deposit or of a characteristic of a portion of the wall inside a fluid vessel.

### BACKGROUND OF RELATED TECHNOLOGY

Deposits of inorganic or organic composition form a fundamental problem as regards the operation of industrial plants in which fluids, particularly aqueous media move through pipe systems or are stored (e.g. intermediately) in containers.

Thus, for example, inorganic deposit of scales can interfere with the operation of refrigerating plants or, in the worst case, cause a total shutdown thereof. Thus, there is a need to have information concerning the existence or the thickness of such an inorganic deposit in a timely manner so that appropriate counter-measures can be taken.

Furthermore, organic deposits often occur in the form of a biofilm. These are formed primarily in aqueous systems at the interface with a solid phase and consist of a slimy layer in which micro-organisms (e.g. bacteria, algae, fungi, and protozoa) are embedded. Biofilms are formed when micro-organisms settle at the interface. As a rule, the biofilm contains, other than the micro-organisms, primarily water and extra-cellular polymeric substances exuded by the micro-organisms which, in conjunction with the water, form hydro-gels and contain other nutrients or substances. Often, particles are included in the resulting slimy matrix that are found in the aqueous medium adjacent the interface. If the conditions for growth are optimal, particularly the temperature and favorable nutrients, then the deposits can grow to a substantial layer thickness. For example, in industrial plants, this can lead to operational errors owing to reduced pipe cross-sections or choking of filters. Moreover, fragments can be released from the biofilm that can affect the operation of the plant. Finally, the material released by the biofilm organisms can additionally accelerate the corrosion of their substrates.

What is particularly problematic is the formation of biofilms in papermaking plants, particularly in the components that are used for the accommodation and transfer of an aqueous fiber suspension. The biofilm (also called "fouling" or "biofouling") which forms in such a papermaking plant is also characterized by the fact that it contains a high proportion of fibers, fine substances, and inorganic pigments that are bound by the organic matrix. Such biofilms typically are accompanied by protective exopolysaccharides ("slime", EPS) and occur at the interface of these equipment surfaces and process water streams. Additionally, inorganic contaminants, such as calcium carbonate ("scale") and organic contaminants often deposit on such surfaces. These organic contaminants are typically known as "pitch" (e.g., resins from wood) and "stickies" (e.g., glues, adhesives, tape, and wax particles).

The biofilm often has a fur-like structure whose thickness can be of the order of a few centimeters. If the layer thickness of the biofilm is too great, it might break away from the substrate. The portions thus released might cause faulty operation, particularly tearing of the paper webs during paper manufacture, which leads to high consequential costs. In order to avoid this, biocides are added, which, however, are expensive and pose a health hazard. Agents which have enjoyed widespread use in such applications include chlorine, organo-mercurials, chlorinated phenols, organo-bromines, and various organo-sulfur compounds, all of which are generally useful as biocides but each of which is attended by a variety of impediments.

It is thus necessary to monitor the layer thickness of the biofilm and possibly the rate of growth thereof in order to optimize the addition of biocides and/or of other deposit control agents.

Furthermore, there can be a tendency of chemical corrosion, cavitation corrosion and generally to corrosive damages of the inner walls of fluid vessels due to the exposure of such inner walls to the fluid environment which might be chemically aggressive. Due to such processes, the wall thickness of the fluid vessel walls can be reduced during the operation and/or times of non-operation of the fluid vessel. It is thus necessary to monitor the thickness of the fluid vessel walls in order to be able to avoid failure of fluid vessels.

There are a variety of devices that have been developed to measure the biofilms in papermaking plants.

One approach provides a disk of Perspex in a container that is connected to the respective component of the paper machines in a bypass and through which the aqueous suspension is passed. The Perspex disk is periodically removed from the liquid by means of a motor and then light beamed through it. The diminution of the light intensity measured is compared with that obtained when there is no deposit. Based on this, conclusions are drawn regarding the thickness of the deposit and its rate of growth. This method suffers from the drawback that the material of the Perspex disk is not the same as that of the containers and pipes, which means that the conditions for growth are not identical. Moreover, the deposit comes into contact with air owing to its periodic withdrawal, which also leads to different growth conditions. In addition, this method can only be used as a bypass and cannot be directly integrated in the pipes or containers of the plant.

A second approach consists in the acquisition of the layer thickness of the biofilm using a light emitting unit that detects and evaluates an optical reflection signal which varies depending on the thickness of the biofilm. The optical method, however, needs an interface with the fluid both for the emitted optical signal and for the reflection signal.

A third approach consists in placing small disks made of stainless steel in the liquid, which are left there for a specific period of time. The deposits on the steel coupons may be subsequently analyzed by microscopy, such as Apotome (AP), Epifluorescence (EP), Scanning Electron (SEM) with EDX, and Confocal Laser Scanning Microscopy (CLSM). These methods are, however, very time-consuming and not suitable for continuous process control.
Furthermore, document DE 102 14 678 A1 discloses a method to determine the thickness of a deposit on the inner walls of fluid containers or pipes by means of ultra sound and document US 2003/0010125 A1 discloses a method and apparatus for analyzing a deposited layer on the inner surface of a fluid container wall. Such methods are not able to determine the distance of an ultrasonic transducer on the one hand and a fluid/deposit interface or a fluid/wall interface on the other hand in an absolute distance measurement with high precision. Document DE 102 14 678 A1 discloses in paragraph [0012] a measurement precision of 1 to 5 millimeters which is insufficient and document US 2003/010125 A1 merely provides a qualitative analysis of the chemical nature of deposited material.
There is a demand for a method that provides continuous online information of the tendency of deposit formation and/or of the tendency of wall thickness reduction or of another characteristic of the fluid vessel wall. Only if the formation of such deposits and/or such wall thickness reduction is detected at an early stage and with high precision (e.g. in the micrometer range), effective and economic counter-measures may be taken, e.g. the addition of suitable deposit control agents or the exchange of the fluid vessel wall, at least partly. The method should allow for an online measurement that may be installed as part of any wall of a vessel or wall of a pipe, i.e., that can be directly integrated in the pipes or containers of the plant. In consequence, the method does not require to be used as a bypass.
It is an object of the invention to provide an alternative device and method of measurement that has advantages when compared with prior art methods.

### SUMMARY OF THE INVENTION

This object is achieved with a method and a device for a high precision measurement of a characteristic of a fouling and/or scaling deposit inside a fluid vessel or of a characteristic of a portion of the wall inside a fluid vessel according to claim 1, wherein the time domain resolution is for example smaller or equal to 700 picoseconds, advantageously smaller or equal to 400 picoseconds, still more advantageously smaller or equal to 200 picoseconds and most advantageously smaller or equal to 120 picoseconds.
It has been surprisingly found by using the device according to the invention that the formation of various deposits in the water can be quantified by means of the method and the device according to the invention for situations that cannot be quantified by conventional plate counts. Further, it has been found that the measurement achieved by cultivation never reaches the amount actually found on the surfaces of the steel coupons. This also shows the necessity of alternative methods for the analysis of the water systems. Online analytical methods are important to be able to take counter-measures when changes of the environment in the fluid vessel or in the fluid bearing system or plant occur over time.

### BRIEF DESCRIPTION OF THE DRAWINGS

Figure 1 is a schematical sectional view of a fluid vessel in the region of an inventive device.
Figures 2a and 2b show schematical sectional views of a fluid vessel in the region of an inventive device according to alternative embodiments.
Figure 3a is a schematic sectional view of a fluid vessel wall in the region of a reflection area with a first kind of deposit.
Figure 3b is a schematic sectional view of a fluid vessel wall in the region of a reflection area with a second kind of deposit.
Figures 4a, 4b and 4c are schematic sectional views of a fluid vessel wall in the region of a reflection area with further kinds of deposit.
Figure 5 is a schematic time-domain diagram showing an emission signal and a first reflection signal.
Figure 6 is a schematic time-domain diagram showing an emission signal and a second reflection signal.
Figure 7 is a schematic time-domain diagram showing an emission signal and a third reflection signal.
Figure 8 is a schematic time-domain diagram showing an emission signal and a fourth reflection signal.
Figures 9 to 14 are schematic sectional or perspective views of different examples for holding a transducer relative to the fluid vessel.
Figure 15 is a schematic sectional view of an experimental setup structure.
Figure 16 is a schematic perspective view of a measuring cell and a deposit plate related to a first example.
Figure 17 is another schematic perspective view of the measuring cell and the deposit plate related to the first example.
Figure 18 is a schematic view of the deposit plate related to a second example.
Figure 19 is another schematic view of the deposit plate related to the second example.
Figure 20 is a schematic view of the deposit plate related to a third example.
Figure 21 is another schematic view of the deposit plate related to the third example.
Figures 22 to 24 show a plurality of different diagrams related to signals from an inventive device.

### DETAILED DESCRIPTION OF THE INVENTION

The present invention relates to a method and a device for a high precision measurement of a characteristic of the fouling and/or scaling deposit inside a fluid vessel or of a characteristic of a portion of the wall inside a fluid vessel by implementing an ultrasonic measurement of the wall thickness or the layer thickness of a fouling and/or scaling deposit layer, i.e. an organic (fouling, biofilm) or inorganic (scaling) deposit or combinations thereof.

When performing the method according to the present invention, a device according to the present invention is located at a fluid vessel where - at the inside surface - a characteristic of the fouling and/or scaling deposit or of a portion of the wall inside the fluid vessel is to be measured.

A fluid is located inside the fluid vessel. The fluid vessel can either be provided completely filled with the fluid or it can be provided only partly filled with the fluid. The fluid vessel is itself part of a fluid bearing system or a plant. For example, the fluid vessel is part of components that serve to accommodate and transfer an aqueous fiber suspension for the manufacture of paper. Other examples for the fluid vessel include tubes or tanks in power plants, components of cooling systems or other fluid bearing systems.

A fluid vessel within the scope of the invention comprises fluid pipes and/or fluid containers, especially those that are used to accommodate and transfer fluids. A fluid vessel in the context of the present invention can be provided, e.g., as a tank, a tower, a pipe, a shower, a channel, a headbox, a pump, a screen, a refiner, a pulper, or a flotation unit.

The fluid or fluids relevant for the present invention can be, e.g., of the following kind: aqueous fluids and/or oleaginous fluids and/or hydrocarbon containing fluids and/or wellbore fluids and/or coolants and/or heating waters and/or industrial waters and/or aqueous fiber suspensions for the manufacture of paper.

The inventive device is preferably directly integrated in the fluid bearing system. However, the inventive device can alternatively be installed in a bypass of the fluid bearing system so as to simplify maintenance of the inventive device. Further, the inventive device can also be provided integrated in the wall of the fluid vessel. For the purpose of the description, the expression "integrated in the wall of the fluid vessel" shall mean that an element is incorporated into the wall of the fluid vessel so that said element has become an integral part of the wall or that a part of said element (especially an inwardly oriented surface) replaces a portion of the fluid vessel wall. For example, an element is to be considered as being integrated in the wall of the fluid vessel, when the wall comprises a cavity, e.g. a hole, and said element has a shape and size that fits into said cavity. Preferably, said element should be incorporated in the wall of the fluid vessel in such a manner that the wall at the location of said element remains tight (with respect to the fluid).

The fluid vessel comprises a fluid vessel wall or (a plurality of) fluid vessel walls. In at least one of the walls of the fluid vessel, an ultrasonic transducer is located. Preferably, the ultrasonic transducer is located such that at least part of the so-called head of the ultrasonic transducer, i.e. a (vibrating) active surface of the ultrasonic transducer, is provided in contact with the fluid inside the fluid vessel. In an alternative embodiment, the ultrasonic transducer is located outside of the fluid vessel wall such that the ultrasonic transducer is not directly in contact with the fluid inside the fluid vessel. The ultrasonic transducer emits an ultrasonic emission signal by means of causing the head of the ultrasonic transducer to vibrate. The ultrasonic emission signal propagates through the fluid in a specific manner which is defined by the form of the head of the ultrasonic transducer, by the excitation mode of the head of the ultrasonic transducer and optionally also by the ultrasonic properties and characteristics of the fluid. The ultrasonic emission signal has a frequency or a center frequency of above 500 kHz, preferably at least 510 kHz, more preferably above 2 MHz, still more preferably at least 2,1 MHz, and most preferably of at least 4 MHz. The ultrasonic emission signal frequency range is in the range from 500 kHz to 50 MHz, preferably in the range from 510 kHz to 50 MHz, more preferably in the range between 2 MHz and 20 MHz, still more preferably in the range from 2,1 MHz to 20 MHz, still more preferably in the range from 3 to 15 MHz, most preferably in the range from 3 MHz to 7 MHz.

In the context of the present invention, it is important that there is at least one region where the ultrasonic emission signal is reflected. In the context of the present invention, this region is called the reflection area. The reflection area is usually located at or near of an area of the wall of the fluid vessel which is located substantially opposite of the transducer. However, the reflection area can also be located within the fluid vessel (e.g. inside a large tube) but attached to a portion of the wall of the fluid vessel, e.g. by means of a holding means. In the context of the present invention, the reflection area can be viewed as being a part of the device, especially for cases where the ultrasonic properties of at least part of the reflection area substantially differ from the ultrasonic properties of the fluid vessel, i.e. from the ultrasonic properties of the (wall) material of the fluid vessel. In such embodiments of the reflection area, there is, e.g., provided an insert in the wall of the fluid vessel such as to alter the ultrasonic properties of the reflection area or a part of the reflection area. Especially for cases where the ultrasonic properties of at least part of the reflection area do not substantially differ from the ultrasonic properties of the fluid vessel, the reflection area is realized as a part of the wall of the fluid vessel.

The ultrasonic emission signal is reflected in the reflection area. According to the present invention, this reflection occurs at the first reflective interface that the ultrasonic wave of the ultrasonic emission signal encounters.

In the case that there is no deposit at the wall of the fluid vessel in the region of the reflection area, the first reflective interface of the ultrasonic wave of the ultrasonic emission signal is the interface of the fluid with the reflection area. The assumption for this case is that there is a substantial difference of the acoustic impedance of the fluid on the one hand and the acoustic impedance of the fluid vessel wall in (at least part of) the region of the reflection area on the other hand. This assumption is, e.g., verified for cases where the fluid is a water-based fluid and the fluid vessel wall material is mainly a metal, especially a steel material. This is because the typical impedance value of a water based fluid is 1,5 MRayl or in the region of 1,5 MRayl and the typical impedance value of metals used as fluid vessel wall material like stainless steel is 40 MRayl or greater than 40 MRayl. The last mentioned assumption is not verified or not necessarily verified, e.g., for the case where the fluid is a water-based fluid and the fluid vessel wall material - at least in the reflection area - is a plastic material, preferably a polypropylene material, having typically an impedance value from 1 MRayl to 4 MRayl, preferably of 2,2 MRayl or around 2,2 MRayl. In a measuring situation or detection situation where a biofouling deposit is to be detected (and its thickness measured) with a water based fluid, it can be preferred to choose to have such a comparably low difference of the acoustic impedance of the fluid on the one hand and the acoustic impedance of the fluid vessel wall in (at least part of) the region of the reflection area on the other hand. With typical values of the acoustic impedance of such biofouling deposits from 1 MRayl to 4 MRayl, it is possible according to the present invention to detect the presence (and measure the thickness) of such a biofouling deposit by means of such a low difference of the acoustic impedance of the fluid on the one hand and the acoustic impedance of the fluid vessel wall in (at least part of) the region of the reflection area on the other hand. In the case, however, that there is a deposit at the wall of the fluid vessel in the region of the reflection area, the first reflective interface of the ultrasonic wave of the ultrasonic emission signal is the interface of the fluid with the deposit in the reflection area, i.e. the interface of the fluid with the top layer or with the "surface of the deposit" in the reflection area. The term "surface of the deposit" is used here to indicate the part of the layer of the deposit that is interacting with the ultrasonic wave of the ultrasonic emission signal in order to provide the first reflective interface. This part of the layer of the deposit that is interacting with the ultrasonic wave of the ultrasonic emission signal is usually a superficial part of the deposit.
In the context of the present invention, the first reflective interface of the ultrasonic wave of the ultrasonic emission signal is also called the fluid/deposit interface or the fluid/wall interface. The term fluid/deposit interface refers to the situation where a deposit (layer) is present in the reflection area. It is evident, that the term "fluid/deposit interface" refers to the interface of the fluid with the deposit in at least part of the reflection area surface. The term fluid/wall interface refers to the situation where a deposit (layer) is not present (or can be neglected with regard to the behavior of ultrasonic waves) in the reflection area but where the fluid directly faces the fluid vessel wall material.
The device is provided such that the signal corresponding to the reflection of the ultrasonic emission signal (hereinafter also called reflective ultrasonic signal) at the fluid/deposit or the fluid/wall interface is acquired. In order to realize this in the most cost-effective manner, the same ultrasonic transducer (that provides the ultrasonic emission signal) is most preferably driven in an acquisition mode such that the ultrasonic wave corresponding to the reflective signal is received by the transducer. In the context of the present invention, the term "substantially opposite" refers to a location of the reflection area and to an inclination of the reflection area such that the ultrasonic emission signal (provided by the ultrasonic transducer) is reflected towards a suitable receiving element for the reflected ultrasonic signal (or the corresponding ultrasonic wave), the receiving element being preferably the ultrasonic transducer. However, according to the present invention, it is possible to use one ultrasonic transducer (uniquely) for providing the ultrasonic emission signal and another ultrasonic transducer (uniquely) for receiving and acquiring the reflective ultrasonic signal. For example, the ultrasonic emission signal can be provided as a (time-domain) pulse signal. This means that the duration of a substantial amplitude of the ultrasonic emission signal is rather limited in time, e.g. considerably less than one millisecond. Furthermore, it is preferred in the context of the present invention that the ultrasonic transducer is used for operation in pulse echo mode (so-called A-scan). This means that the ultrasonic emission signal has finished at the time of arriving of the reflective ultrasonic signal. It is most preferred that the features of the ultrasonic emission signal being provided as a pulse signal on the one hand and of the ultrasonic transducer being used for operation in pulse echo mode on the other hand are combined. This allows for a comparably simple data acquisition and data analysis. The ultrasonic emission signal being a (time-domain) pulse signal implies that the frequency of the emission signal is a single frequency. According to the present invention, the term "frequency of the emission signal" is used to refer to a meaningful frequency, i.e. characterizing the emission signal, such as for example the center frequency of the emission signal.

One of the main aspects of the present invention is to provide a cost-effective possibility of a high precision measurement of the distance between the ultrasonic transducer and the fluid/deposit or fluid/wall interface. When assuming a constant speed of sound throughout the fluid between the ultrasonic transducer and the fluid/deposit or fluid/wall interface, the distance to be measured corresponds either to the time required for the ultrasonic emission signal travelling from the ultrasonic transducer to the fluid/deposit or fluid/wall interface and to the time required for the reflective ultrasonic signal (corresponding to the reflection of the ultrasonic emission signal at the fluid/deposit or fluid/wall interface) travelling from the fluid/deposit or fluid/wall interface back to the ultrasonic transducer. Therefore, the distance (to be measured) corresponds to the speed of sound in the fluid multiplied by one half of the time delay between corresponding parts of the ultrasonic emission signal on the one hand and the reflective ultrasonic signal on the other hand. The reflective ultrasonic signal received by the transducer will usually have a waveform (or amplitude envelope) that differs from the waveform (or amplitude envelope) of the ultrasonic emission signal due in part to the reflection at the fluid/deposit or fluid/wall interface but also due to other effects like diffraction and the like. According to the present invention, the ultrasonic transducer acquires the reflective signal received and determines the time of arrival of the reflective signal (and by means of the time of arrival also the time delay between the time of emission of the ultrasonic emission signal and the time of arrival of the reflective ultrasonic signal). According to the present invention, the reflective signal is assumed to have arrived if the strength (i.e. the absolute amplitude) of the reflective signal rises above a suitable threshold value (suitable threshold values being above the average level of noise). The time of arrival of the reflective signal can be deduced from that rise above the threshold value. According to the present invention, the time-domain resolution power of the determination of the time delay between the time of emission of the ultrasonic emission signal and the time of arrival of the reflective ultrasonic signal is 1 ns or less than 1 ns. Preferably, this time-domain resolution is smaller or equal to 700 picoseonds, more preferably smaller or equal to 400 picoseconds, still more preferably smaller or equal to 200 picoseconds and most preferably smaller or equal to 120 picoseconds.

According to the present invention, a high precision measurement of a characteristic of the fouling and/or scaling deposit inside the fluid vessel or of a characteristic of a portion of the wall inside the fluid vessel is performed. As one main example of such a characteristic of the deposit is the thickness of the deposit layer (or the remaining thickness of the wall material) in the reflection area, the distance between the ultrasonic transducer and the fluid/deposit or fluid/wall interface is measured. In this context, a high precision measurement means that it is possible to determine the characteristic wih a comparably high relative accuracy, e.g. a relative accuracy of less than 0,0015, preferably of less than 0,0010, more preferably of less than 0,0005, still more preferably of less than 0,0001 and most preferably of less than 0,00005. According to the present invention, the distance between the ultrasonic transducer and the fluid/deposit or fluid/wall interface can be measured with an accuracy of +/-15 micrometer, preferably with an accuracy of +/- 10 micrometer, more preferably with an accuracy of +/- 7 micrometer, still more preferably with an accuracy of +/- 5 micrometer, even more preferably with an accuracy of +/- 3 micrometer and most preferably with an accuracy of +/- 1 micrometer. With the information related to the distance between the ultrasonic transducer and the fluid/deposit or fluid/wall interface, it is possible to determine or to calculate the thickness of the deposit layer (by means of measuring the distance between the ultrasonic transducer and the fluid/deposit or fluid/wall interface without any deposit or by means of the dimensions of the fluid vessel). For example, for opposing walls of the fluid vessel spaced by 10 millimeter (or for a distance between the ultrasonic transducer and the reflection area of 10 millimeter, a relative accuracy of 0,0015 corresponds to a measurement accuracy of +/- 15 micrometer. For greater absolute distances, a relative accuracy of less than 0,0015, preferably of less than 0,0010, more preferably of less than 0,0005, still more preferably of less than 0,0001 and most preferably of less than 0,00005 is possible with the device and the method of the present invention. Preferred distances between the ultrasonic transducer and the reflection area according to the present invention are in the range from 10 millimeter to 500 millimeter, preferably in the range from 10 millimeter to 200 millimeter, more preferably in the range from 10 millimeter to 100 millimeter, most preferably in the range from 20 millimeter to 50 millimeter. Additionally the inventive method and device may allow for the measurement of at least a further characteristic of the deposit (besides the measurement of the distance between the ultrasonic transducer and the fluid/deposit or the fluid/wall interface) formed during an ongoing process. For example, the measurement of the further characteristic is preferably conducted by means of evaluating the time-domain reflective signal of the fluid/deposit or fluid/wall interface. The further characteristic of the deposit is preferably the density and/or the homogeneity and/or the stability of the deposit or of the fluid vessel wall. These further characteristics of the deposit layer or of the fluid vessel wall may be monitored simultaneously or may be deduced by analyzing a multitude of different measurements. Variations in the layer density of the deposit or of the fluid vessel wall and layer homogeneity of the deposit or of the fluid vessel wall also result in differences with respect to the reflective signal (i.e. the reflection of the ultrasonic emission signal at the fluid/deposit or fluid/wall interface). Based on the set of information provided by the measurement, especially a multitude of measurements, it may also be concluded which particular type of deposit has been formed or if there have been corrosive damages on the fluid vessel inside wall surface (in the reflection area). In consequence, the appropriate deposit control agent (or a fluid vessel wall curing agent) may be selected and added in order to initiate suitable countermeasures without interrupting the process. The effect of such a control agent can be observed and if necessary, its concentration, location of supply, chemical composition, etc. may be changed and adjusted, respectively.

According to the method and device according to the present invention, the temperature of the fluid is measured and the distance between the ultrasonic transducer on the one hand and the fluid/deposit interface or the fluid/wall interface on the other hand is derived from the arrival time of the reflective signal and the speed of sound of the fluid, the speed of sound being dependent on the temperature of the fluid. According to another preferred embodiment, the ultrasonic transducer is provided in contact with the fluid or in contact with the inside of the fluid vessel. In a still more preferred embodiment of the method and device according to the present invention, both the temperature of the fluid is measured, and the ultrasonic transducer is provided in contact with the fluid or in contact with the inside of the fluid vessel.
In the embodiments of the present invention, provision is made for a compensation of temperature effects that would otherwise reduce the overall accuracy. Accordingly, the inventive device therefore provides at least a temperature compensation when calculating, e.g., the distance between the ultrasonic transducer and the fluid/deposit or fluid/wall interface. The temperature compensation is conducted by a measuring step, preferably by directly measuring the temperature of the fluid with a temperature sensor, e.g. in an area adjacent to or integrated with the active surface (ultrasonic transducer head) of the ultrasonic transducer. In a second step, the compensation is calculated by means, e.g., of data values that assign a certain speed of sound of the fluid used to a certain temperature. An analogous temperature compensation can be done for the changes in dimensions of the fluid vessel (or fluid vessel walls), e.g. changing diameters or the like, as a function of different temperatures.

The method and device for a high precision measurement of a characteristic of the fouling and/or scaling deposit inside a fluid vessel or of a characteristic of a portion of the wall inside the fluid vessel according to any embodiment of the present invention may be used as or in connection to an online (monitoring) device. Preferably, the measurements are performed on stainless steel substrates or on other vessel materials on which deposits have formed. The goal of the inventive method and device is to determine as much data as possible to make the relevant decisions in view of injecting descaling and/or antifouling agents into the system before any disturbances occur in the process, e.g. a process for making paper or a cooling or heating process. Such an online monitoring device that can be directly built into the process gives a possibility to gain reliable data, where the changes in flow etc. are not factors for any false readings.
The method and device for a high precision measurement of a characteristic of the fouling and/or scaling deposit inside a fluid vessel or of a characteristic of a portion of the wall inside the fluid vessel according to any embodiment of the present invention is suitable as long as there is a fluid/deposit or fluid/wall interface providing a sufficiently strong ultrasonic reflection signal, i.e. with a sufficiently strong signal to noise ratio. This is particularly the case with inorganic scale in cooling plants, especially in cooling towers of power plants; deposits occurring in devices used for the processing of food or beverages, such as breweries, e.g., screw extruders, meat grinders, and the like; deposits occurring in devices used for waste-water treatment; deposits occurring in devices used in exhaust gas purification; deposits occurring in devices used in mining industry or oil production. For the measurement at the fluid/deposit interface of fouling deposits, the change in acoustic impedance (compared to the acoustic impedance of the fluid) is often (especially in the case of an aqueous fluid) reduced compared to the case of an inorganic deposit. According to a specific aspect of the present invention, the inventive device provides for an adaptation of the material of at least part of the reflection area such that a reflection at the interface of the deposit and the fluid vessel wall (or at the interface of the deposit and a holder (or holding means) of the reflection area) is reduced. This can be realized, e.g., by means of providing a polyurethane or a polypropylene surface (or a surface of another polymeric material or plastic material) in at least part of the reflection area. Such a polyurethane or polypropylene or polymeric or plastic surface has the effect that ultrasonic waves hitting such a surface are absorbed to a comparably high degree (comparably high transmission coefficient at the interface between of the fluid with the polyurethane or polypropylene or polymeric or plastic surface and comparably high energy dissipation of acoustic energy inside of the polyurethane or polypropylene or polymeric or plastic material) and that the presence of an organic (fouling) deposit on such a polyurethane or polypropylene or polymeric or plastic surface in at least part of the reflection area leads potentially to a higher degree of reflection (based, e.g., on a higher change in acoustic impedance at the fluid/deposit interface). The resulting effect is that what is detected when evaluating the reflective signal is indeed the reflection at the fluid/deposit interface (and not the reflection at the interface of the deposit on the one hand and the fluid vessel wall (or a holder of the reflection area) on the other hand). In the case of an organic deposit on a fluid vessel wall made, e.g., of steel in the relevant part of the reflection area and despite the presence of a deposit (having an acoustic impedance comparable to the acoustic impedance of the fluid), this could be more difficult, as the reflective signal could relate to the interface of the deposit with the fluid vessel wall (deposit/wall interface) rather than to the fluid/deposit interface.

The kinds of organic deposits (fouling or biofouling) detectable with the method and device of the present invention are all kinds of biofilms, e.g. those occurring in papermaking plants or those occurring in water bearing systems and potentially leading to the growth of bacteria like the bacteria of the family of Legionellaceae, especially Legionella. For the purpose of the description, the term "biofilm" or "fouling" preferably refers to all kinds of slimy layers in which micro-organisms (e.g. bacteria, algae, fungi, and protozoa) are embedded. Said biofilms may contain, other than the micro-organisms, primarily water, extra-cellular polymeric substances exuded by the micro-organisms which, in conjunction with the water, form hydro-gels and contain other nutrients, or substances, such as fibers, fines, inorganic pigments, particularly scale, aluminum hydroxide (alum addition in neutral pH-range), natural and synthetic substances, like pitch, fatty acids, sizing agents (alkenylsuccinic anhydride [ASA], alkylketene dimer [AKD], rosin size, etc.), white pitch, and so called "stickies" (polyacrylates, polyvinylacetate, ethylene vinylacetate, styrene butadiene latex, etc.).For the purpose of the specification the term "biofilm" or "fouling" preferably refers to all layers that may have been deposited on each other in a biofilm.

Various deposit control agents have been developed which may be added to a water bearing system or to a papermaking plant during operation.

The method and device according to the present invention allow for online monitoring of the absolute distance between the ultrasonic transducer at a wall of the fluid vessel on the one hand and a fluid/deposit or a fluid/wall interface on the other hand, wherein the fluid/deposit or fluid/wall interface is located in the reflection area and wherein the reflection area is located substantially opposite of the ultrasonic transducer. Thereby, it is possible according to the present invention to provide a high precision measurement of the thickness (or the layer thickness) of the deposit or of the fluid vessel wall located in the reflection area of the ultrasonic transducer.

According to the present invention, it is preferred that the measurements of the thickness of the deposit, i.e. the measurements of the absolute distance between an ultrasonic transducer at a wall of the fluid vessel on the one hand and fluid/deposit or fluid/wall interface on the other hand is repeated comparably often, for example at least once in every minute, preferably at least once in every second, more preferably at least once in every 100 milliseconds and still more preferably at least once in every 10 milliseconds and most preferably every 1 to 6 milliseconds. As the measurement of the absolute distance between an ultrasonic transducer at a wall of the fluid vessel on the one hand and fluid/deposit or fluid/wall interface on the other hand, and hence the measurement of the thickness of the deposit (or the (remaining) thickness of the wall), is repeated comparably often, also the rate of growth of the deposit (or the rate of the reduction of the thickness of the fluid vessel wall) can be deduced from a plurality of measurements, by monitoring the changes over a certain period of time.

The present invention is well suited for studying the growth of biological materials and the deposition of organic and inorganic contaminants on various substrates. Such biological materials include, for example, bacteria, fungi, yeast, algae, diatoms, protozoa, macroalgae, and the like. In the pulp and paper industry, process water provides an excellent supply of organic and inorganic materials which promote the growth of bacteria (biofilms) and protective exopolysaccharides (slime) which occur at the interface of machine surfaces (typically steel) and process water streams. Additionally, inorganic contaminants, such as calcium carbonate ("scale") and organic contaminants often deposit on such surfaces. These organic contaminants are typically known as pitch (e.g., resins from wood) and stickies (e.g., glues, adhesives, tape, and wax particles).

It has been surprisingly found that the measuring system according to the invention overcomes the disadvantages of the prior art and provides a cost-effective solution for the acquisition of the thickness (or layer thickness) of a deposit, particularly an inorganic (scale) deposit or a biofilm (or fouling deposit). By means of the method and device according to the present invention, it is possible to measure the thickness of (mostly) inorganic deposits as well as of fouling deposits and also of those fouling deposits having a fur-like structure such as occur in papermaking plants. Furthermore, by means of the method and device according to the present invention, it is possible to measure the thickness of combined organic and inorganic deposit layers. Furthermore, it is possible to measure not only the thickness of the deposit and its rate of formation, but also its density, its homogeneity and/or its stability, in order to get further insights into the deposition processes taking place thereby providing the information that is needed for initiating appropriate counter-measures. Furthermore, by means of the method and device according to the present invention, it is possible to measure a characteristic, especially the thickness, of the wall inside a fluid vessel in order to get information on chemical corrosion, cavitation corrosion and generally to corrosive damages of the inner walls of the fluid vessel.

According to the present invention, it is preferred that in addition to measuring the time-domain reflective signal of the fluid/deposit or fluid/wall interface, the change of the acoustic impedance at the fluid/deposit or fluid/wall interface is measured. This can be done by means of comparing the amplitudes of the ultrasonic emission signal and the ultrasonic reflective signal. As the reflection coefficient of the ultrasonic wave at the fluid/deposit or fluid/wall interface is proportional to the ratio of the difference of the acoustic impedances at both sides of the interface on the one hand and the sum of the acoustic impedances at both sides of the interface on the other hand, a comparably strong ultrasonic reflective signal indicates a comparably strong difference of the acoustic impedance at both sides of the fluid/deposit or fluid/wall interface. Such a comparably strong difference of the acoustic impedances at both sides of the fluid/deposit or fluid/wall interface indicates, e.g., a certain nature of the deposit on the inner fluid vessel wall, e.g. an inorganic (scale) deposit rather than a biofilm. However, a comparably weak reflection signal (i.e. the maximum amplitude of the reflection signal and/or the integral of the reflection signal) does not necessarily indicate a comparably weak difference of the acoustic impedances at both sides of the fluid/deposit or fluid/wall interface, due to other effects like scattering or the like.

According to the present invention, it is preferred that a characteristic of the fouling and/or scaling deposit on the reflection area or a characteristic of the wall in the reflection area is derived by means of the determination of the evolution of the change of the acoustic impedance at the fluid/deposit or fluid/wall interface. By means of measuring repeatedly the characteristic of a fouling and/or scaling deposit inside the fluid vessel or of a characteristic of a portion of the wall inside the fluid vessel, it is possible to derive further insights in the formation process of the deposit or the corrosive process at the fluid vessel wall. Especially, it is possible according to the present invention to distinguish, e.g., the situation of a growing biofouling deposit layer from the situation of a growing (inorganic) scale deposit layer by means of the evolution of the change of the acoustic impedance at the fluid/deposit or fluid/wall interface. The acoustic impedance of a scale deposit layer has typically a value in the range from 10 MRayl to 30 MRayl. The acoustic impedance of a biofouling deposit layer has typically a value in the range from 1 MRayl to 2 MRayl. By means of monitoring the evolution of the change of the acoustic impedance at the fluid/deposit or fluid/wall interface, it is possible to detect the growth of a biofouling deposit or of a scale deposit. For the case of a metal as fluid vessel wall material in the reflection area, such as stainless steel, a growing scale deposit layer results in a comparably strong reduction of the acoustic impedance at the fluid/deposit interface or fluid/wall interface. For the case of a metal as fluid vessel wall material in the reflection area, such as stainless steel, a growing biofouling deposit layer results in an almost unchanged acoustic impedance at the fluid/deposit interface or fluid/wall interface. For the case of a metal as fluid vessel wall material in the reflection area, such as stainless steel, a growing combined scale/biofouling deposit layer results in a reduction of the acoustic impedance at the fluid/deposit interface or fluid/wall interface. For the case of a plastic as fluid vessel wall material in the reflection area, such as polypropylene, a growing scale deposit layer results in a comparably strong increase of the acoustic impedance at the fluid/deposit interface or fluid/wall interface. For the case of a plastic as fluid vessel wall material in the reflection area, such as polypropylene, a growing biofouling deposit layer results in a comparably weak reduction of the acoustic impedance at the fluid/deposit interface or fluid/wall interface. For the case of a plastic as fluid vessel wall material in the reflection area, such as polypropylene, a growing combined scale/biofouling deposit layer results in an increase of the acoustic impedance at the fluid/deposit interface or fluid/wall interface.

According to the present invention, it is preferred that the thickness of the deposit and/or the build-up of the deposit is measured by means of an initially comparably strong first reflection signal corresponding to a comparably thin layer of the deposit and an at least transiently weaker second reflection signal corresponding to a comparably thick layer of the deposit, and wherein the deposit is preferably a substantially inorganic scale deposit.

Furthermore, it is preferred that the fluid has a first acoustic impedance for ultrasonic waves in the range from 1400 Ns/m³ to 1800 Ns/m³ and the reflection area has at least partly a second acoustic impedance for ultrasonic waves, wherein
a) the second acoustic impedance exceeds the first acoustic impedance by at least 50%, preferably by at least 100%, more preferably by at least 200%, still more preferably by at least 500% and most preferably by at least 900%,
   or wherein
b) the first acoustic impedance exceeds the second acoustic impedance by at least 40%, more preferably by at least 70%, still more preferably by at least 100% and most preferably by at least 150%.

For example, it is thereby possible to determine the formation of, e.g., a craggy surface of the deposit as this can often be seen when inorganic scale deposits begin to grow. The relatively weak second reflection signal corresponds to a comparably low degree of reflection and to a relatively high degree of scattering effects of the ultrasonic emission signal at the fluid/deposit or fluid/wall interface.

According to the present invention, it is preferred that the thickness of the deposit and/or the build-up of the deposit is measured by means of an initially comparably weak third reflection signal corresponding to a comparably thin layer of deposit and an at least transiently stronger fourth reflection signal corresponding to a comparably thick layer of the deposit, and wherein the deposit is preferably a substantially organic biofouling deposit.

Furthermore, it is preferred that the fluid has a first acoustic impedance for ultrasonic waves in the range from 1400 Ns/m³ to 1800 Ns/m³ and the reflection area has at least partly a third acoustic impedance for ultrasonic waves, wherein
a) the third acoustic impedance exceeds the first acoustic impedance by 5% to 50%, preferably by 8% to 30%, more preferably by 10% to 20%,
   or wherein
b) the first acoustic impedance exceeds the third acoustic impedance by 5% to 50%, preferably by 8% to 30%, more preferably by 10% to 20%.
For example, it is thereby possible to determine the formation of, e.g., a biofilm or fouling deposit present on a polyurethane or polypropylene or polymeric or plastic surface (having the property of an acoustic impedance corresponding to the third acoustic impedance). The relatively strong fourth reflection signal corresponds to a comparably high degree of reflection and to a relatively low degree of scattering effects of the ultrasonic emission signal at the fluid/deposit interface.

In an embodiment of the present invention, where at least part of the reflection area is realized by means of a material having the third acoustic impedance, it is preferred that this is realized by means of a polyurethane or polypropylene or polymeric or plastic material. Such a material is comparably inert to a multitude of fluids and can be provided with an adapted acoustic impedance.

According to the present invention, it is preferred that the reflection area comprises a first part and a second part, wherein the acoustic impedance of the first part of the reflection area corresponds to the second acoustic impedance, and wherein the acoustic impedance of the second part of the reflection area corresponds to the third acoustic impedance. Thereby, it is advantageously possible to simultaneously measure in parallel the formation of an organic and the formation of an inorganic deposit layer.
According to still another preferred embodiment of the present invention, the device comprises first ultrasonic transducer and a second ultrasonic transducer, the first ultrasonic transducer being located substantially opposite of the first part of the reflection area and the second ultrasonic transducer being located substantially opposite of the second part of the reflection area. Thereby, it is advantageously possible to simultaneously measure in parallel the formation of an organic and the formation of an inorganic deposit layer. A control unit can be assigned to the device, the control unit being preferably located inside a housing common to the device, and that the control unit is preferably configured to acquire the time-domain reflective signal of the fluid/deposit or fluid/wall interface and to generate a signal related to the thickness of the deposit or of the wall. Thereby, it is advantageously possible to provide a direct measure of the thickness of the deposit layer (or of the wall thickness) by means of a absolute value measurement. It can be preferred that an evaluation module is either integrated in the control unit or assigned to the control unit. The control unit and/or the evaluation module can contain a conventional type of memory. Preferably, the evaluation module can produce a deposit control signal based on the measure of the thickness of the deposit layer and based on a threshold value, the threshold value being either calculated from other measured signals and/or values or predetermined and stored in a memory. The deposit control signal can be used to control an input unit for a biocide or another deposit control agent so that the addition of expensive substances posing a health hazard can be optimized.

A further aspect of the invention relates to the use of the device according to the invention, as described above, corresponding to one of the above-mentioned embodiments, for a high precision measurement of a characteristic of the fouling and/or scaling deposit inside a fluid vessel.

The inventive method and device can preferably be used for a high precision measurement of a characteristic of the fouling and/or scaling deposit inside a fluid vessel or of a characteristic of a portion of the wall inside a fluid vessel of a fluid bearing system of any kind, e.g. in a papermaking plant and/or a power plant and/or a heat transfer assembly and/or a refrigerating plant and/or a waste water effluent and/or a membrane purification system and/or a reverse osmosis filtration unit and/or a ultrafiltration unit and/or a sand filter and/or a steam generating system and/or a boiler and/or a heat exchanger and/or an evaporative condenser and/or a cooling tower and/or a cooling water system and/or a closed cooling system and/or an air washer and/or a device for heating and/or a ventilating and air conditioning (HVAC) device and/or a pasteurizer and/or a sterilizer and/or an engine and/or a biodiesel plant and/or an oil separator and/or a medical device and/or a device for processing food. Very specifically, the fluid bearing system is preferably a component of a papermaking plant which serves the purpose of accommodating or transferring an aqueous fiber suspension employed in papermaking. The fluid bearing system can also be provided as an industrial water system. Such industrial water systems include, but are not limited to, cooling tower water systems (including open recirculating, closed and once-through systems); petroleum wells, downhole formations, geothermal wells and other oil field applications; boilers and boiler water systems; mineral process waters including mineral washing, flotation and benefaction; paper mill digesters, washers, bleach plants and white water systems; black liquor evaporators in the pulp industry; gas scrubbers and air washers; continuous casting processes in the metallurgical industry; air conditioning and refrigeration systems; industrial and petroleum process water; indirect contact cooling and heating water, such as pasteurization water; water reclamation and purification systems; membrane filtration water systems; food processing streams (meat, vegetable, sugar beets, sugar cane, grain, poultry, fruit and soybean); and waste treatment systems as well as in clarifiers, liquid-solid applications, municipal sewage treatment and industrial or municipal water systems.

A further subject of the present invention relates to a method for monitoring a characteristic of the fouling and/or scaling deposit or a characteristic of a portion of the wall, comprising the step of measuring the thickness of the deposit or of the wall by means of the device as described above, corresponding to one of the above-mentioned embodiments.

With respect to such a method for monitoring the characteristic of the fouling and/or scaling deposit inside a fluid vessel, it is preferred according to the present invention, that the fluid bearing system comprises a regulating unit for a deposit control agent and that the method further comprises the steps of:
a) measuring the thickness of the deposit with the device to generate a measuring signal depending on the accumulation of the deposit inside the fluid vessel; and
b) adjusting the supply of a deposit control agent by means of the regulating unit dependent on a comparison made between the measured thickness of the deposit and a threshold value of the thickness of the deposit,
wherein the threshold value is preferably either predefined threshold value or a calculated threshold value. If the measurement in step a) reveals that the measured thickness of the deposit is higher than the threshold value (and/or that another measured and/or calculated characteristics of the deposit compared to a corresponding threshold value is outside of a normal range), the regulation unit may be adjusted and/or actuated in that the supply of deposit control agent will be e.g. increased, and if the measured thickness of the deposit is lower than the threshold value, the regulation unit may be adjusted in that the supply of deposit control agent will be e.g. decreased, and vice versa. In a preferred embodiment of such a method according to the invention, the measured values of the thickness of the deposit (or an average of a multitude of measurements conducted during, e.g., 0,1 seconds to one hour) are stored in a suitable memory. Preferably, a data point is stored at least every hour, more preferably at least every 30 minutes, still more preferably at least every 15 minutes, most preferably at least every 5 minutes and in particular at least every 60 seconds. The data point that is stored may be the average value of several individual data points measured over a time interval that is shorter than the period of time between the storage of given data point in the memory and the storage of the subsequent data point. Since organic deposits, e.g. fur-like films, form in such plants and since these are difficult to measure with conventional measuring systems, the invention now presented at least represents a useful alternative.

Preferably, within the fluid bearing system, the regulating unit is located upstream with respect of the measuring system so that at least a portion of the deposit control agent that is fed into the fluid that passes the measuring system. The regulating unit may be any device that is suitable to add deposit control agents, solids or liquids, to the fluid bearing system in a controlled, dosed fashion. Examples of suitable regulating units include automatic spray units, dropping funnels, injectors, syringes, and the like. The regulating unit feeds the deposit control agents into the fluid bearing system where the deposit is forming or is most likely to form in order to impede the formation of deposits and/or to remove deposits that have already formed. In a preferred embodiment of the process according to the invention, the regulating unit for the deposit control agent is located at the inlet of a fluid stream to a chest, tower or fluid loop, and the measuring system is located at the outlet of the chest, tower or fluid loop.

Other preferred embodiments of the method and device according to the invention are illustrated by the variants displayed in the exemplary embodiment. The invention is described in further detail below with reference to exemplary embodiments and to the associated drawings.

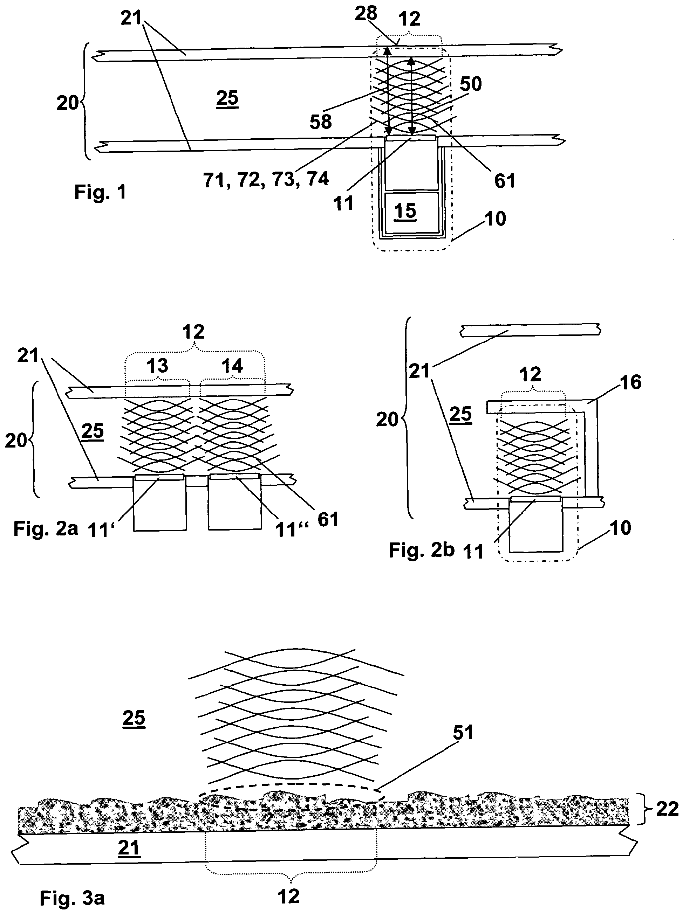

Figure 1 shows a schematical sectional view of a fluid vessel 20 in the region of a first embodiment of an inventive device 10 for a high precision measurement of a characteristic of the fouling and/or scaling deposit inside the fluid vessel 20 or of a characteristic of a portion of the wall 21 inside the fluid vessel 20. A fluid 25 is located inside the fluid vessel 20.

The fluid vessel 20 comprises fluid vessel walls 21. In one of the walls 21 of the fluid vessel 20, an ultrasonic transducer 11 is located. Preferably, the ultrasonic transducer 11 is located such that at least part of the so-called head of the ultrasonic transducer 11, i.e. a (vibrating) active surface of the ultrasonic transducer 11, is provided in contact with the fluid 25 inside the fluid vessel 20. The ultrasonic transducer 11 emits an ultrasonic emission signal 61 by means of causing the head of the ultrasonic transducer 11 to vibrate. The ultrasonic emission signal 61 propagates through the fluid 25 in a specific manner which is defined by the form of the head of the ultrasonic transducer 11, by the excitation mode of the head of the ultrasonic transducer 11 and optionally also by the ultrasonic properties and characteristics of the fluid 25.

The ultrasonic emission signal 61 is reflected in at least one region. In the context of the present invention, this region is called the reflection area 12. The reflection area 12 is usually located at or near of an area of the wall 21 of the fluid vessel 20 which is located substantially opposite of the transducer 11 but the reflection area 12 can also be held or attached by means of a holding means at a certain distance to the wall of the fluid vessel 20 (cf. Figure 2b).

The ultrasonic emission signal 61 is reflected in the reflection area 12. According to the present invention, this reflection occurs at the first reflective interface that the ultrasonic wave of the ultrasonic emission signal 61 encounters. In the case that there is no deposit 22 in the region of the reflection area 12, the first reflective interface of the ultrasonic wave of the ultrasonic emission signal 61 is the interface of the fluid 25 with the reflection area 12, also called fluid/wall interface. In the case, however, that there is such a deposit 22 in the region of the reflection area 12, the first reflective interface of the ultrasonic wave of the ultrasonic emission signal 61 is the interface of the fluid 25 with the deposit 22 on the reflection area 12, i.e. the interface of the fluid 25 with the top layer or with the "surface" of the deposit 22 on the reflection area 12. The term "surface" of the deposit 22 is used here to indicate the part of the layer of the deposit 22 that is interacting with the ultrasonic wave of the ultrasonic emission signal 61 in order to provide the first reflective interface. In the context of the present invention, the first reflective interface of the ultrasonic wave of the ultrasonic emission signal 61 is also called the fluid/deposit or fluid/wall interface 51. It is evident, that the term "fluid/deposit interface" refers to the interface of the fluid 25 with the deposit 22 on the reflection area 12. The term fluid/wall interface refers to the situation where a deposit (layer) 22 is not present (or can be neglected with regard to the behavior of ultrasonic waves) in the reflection area 12 but where the fluid 25 directly faces the fluid vessel wall 21 material.

The device 10 is preferably provided such that a reflective ultrasonic signal (i.e. a signal corresponding to the reflection of the ultrasonic emission signal 61) is acquired by the ultrasonic transducer 11. In the example shown in the Figures, the ultrasonic emission signal 61 is provided as a (time-domain) pulse signal and the ultrasonic transducer 11 is used for operation in pulse echo mode. This allows for a comparably simple data acquisition and data analysis.

One of the main aspects of the present invention is to provide a cost-effective possibility of a high precision measurement of the distance between the ultrasonic transducer 11 and the fluid/deposit or fluid/wall interface 51. In Figure 1 this distance between the ultrasonic transducer 11 and the fluid/deposit or fluid/wall interface 51 is represented by an arrow and the reference numeral 50. The distance 50 corresponds (assuming a constant speed of sound throughout the fluid 25 between the ultrasonic transducer 11 and the fluid/deposit or fluid/wall interface 51) either to the time required for the ultrasonic emission signal 61 traveling from the ultrasonic transducer 11 to the fluid/deposit or fluid/wall interface 51 or to the time required for the reflective ultrasonic signal travelling from the fluid/deposit or fluid/wall interface 51 back to the ultrasonic transducer 11. Therefore, the distance 50 corresponds to the speed of sound in the fluid 25 multiplied by one half of the time delay between corresponding parts of the ultrasonic emission signal 61 on the one hand and the reflective ultrasonic signal on the other hand.

In order to provide a cost-effective possibility of a high precision measurement of the distance between the ultrasonic transducer 11 and the fluid/deposit or fluid/wall interface 51, it is necessary to be able to compensate variations of certain additional parameters such as the temperature of the fluid 25. In the context of the present invention, it is possible to provide information about these additional parameters by means of one additional sensor or a plurality of additional sensors. In an alternative embodiment of the present invention, it is also possible to measure a distance 58 between the ultrasonic transducer 11 and an outer wall surface 28 (e.g. of the fluid vessel wall 21) in an absolute distance measurement by means of evaluating the time-domain reflective signal of the outer wall surface 28. Particularly, it is advantageous in the context of the present invention to deduce the temperature of the fluid 25 from the evaluation of this distance 58 between the ultrasonic transducer 11 and an outer wall surface 28.

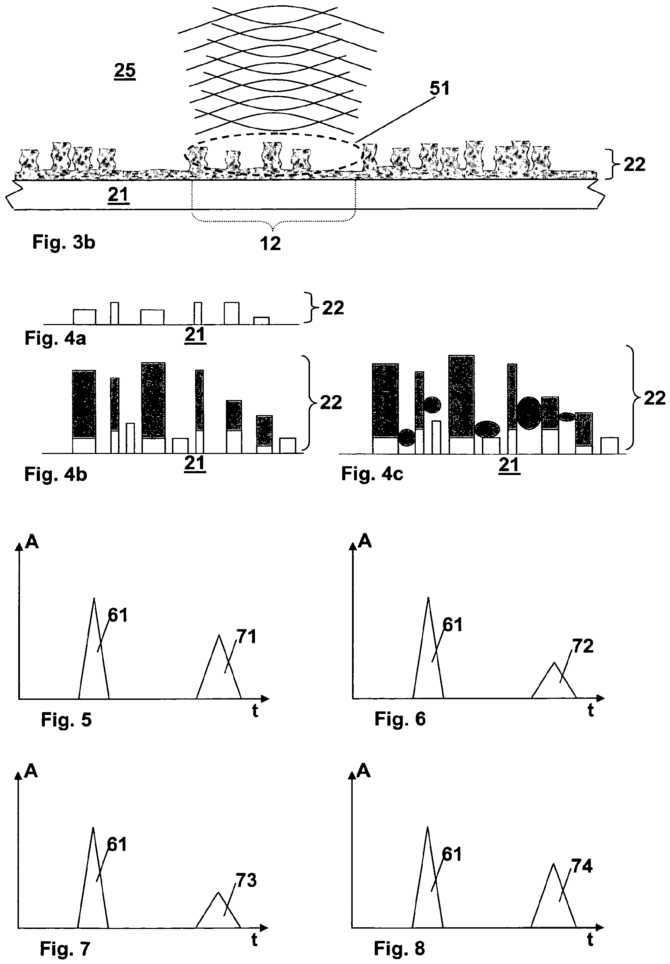

In the context of the present invention, the reflective ultrasonic signal is represented by reference numerals 71, 72, 73 and 74 and is referred to as the first reflection signal 71, the second reflection signal 72, the third reflection signal 73 and the fourth reflection signal 74. These terms are further explained in connection with Figures 5 to 8.

Figures 2a and 2b show schematical sectional views of the fluid vessel 20 in the region of the inventive device 10 according to alternative embodiments of the present invention. In the alternative embodiment shown in Figure 2a, the reflection area 12 comprises a first part 13 and a second part 14. In the first part 13 of the reflection area 12, the reflection area 12 has the property of the second acoustic impedance and in the second part 14 of the reflection area 12, the reflection area 12 has the property of the third acoustic impedance. Compared to the first acoustic impedance which corresponds to the acoustic impedance of the fluid 25, the second acoustic impedance is substantially different, i.e. it differs from the first acoustic impedance by at least 50%, preferably at least 100%, more preferably by at least 200%, still more preferably by at least 500% and most preferably by at least 900%. Compared to the first acoustic impedance, the third acoustic impedance is substantially equl, i.e. it differs from the first acoustic impedance by 5% to 100%, preferably by 8% to 30%, more preferably by 10% to 20%. In the case of the second embodiment, the first part 13 and the second part 14 are preferably made of different materials, one of them for example of the material of the fluid vessel 20 and the other of a different material, provided, e.g., by means of an insert into the wall 21 of the fluid vessel 20.

It is preferred in the alternative embodiment shown in Figure 2a that there is a first ultrasonic transducer 11' substantially opposite of the first part 13 of the reflection area 12 and a second ultrasonic transducer 11" substantially opposite of the second part 14 of the reflection area 12. However, according to the present invention, it is also possible to realize the reflection area 12 with a first and a second part 13, 14 with only one (unique) ultrasonic transducer 11, e.g. by means of using a different actuation mode and by ensuring that at least part of the signal reflected from the fluid/deposit interface 51 is reflected to such a unique ultrasonic transducer 11.

In the alternative embodiment shown in Figure 2b, the reflection area 12 is held or is attached to the wall by means of a holder or a holding means 16. The reflection area 12 can have the property of the second acoustic impedance or of the third acoustic impedance. The holding means 16 can either be separated from the device 10 (shown in Figure 2b) or can be included in the device 10.

In Figure 3a, a schematic sectional view of the wall 21 of the fluid vessel 20 in the region of the reflection area 12 with a first kind of deposit 22 is shown. In Figure 3b, a schematic sectional view of the wall 21 of the fluid vessel 20 in the region of the reflection area 12 with a second kind of deposit 22 is shown. The first and second kind of deposit 22 shown in Figures 3a and 3b correspond to structures than can be easily monitored by the inventive device. With increasing layer thickness the deposit pieces tend to be partially or totally released from the surface they adhere to. The stability of the deposit is also highly affected by the water content. The measuring system provides information about the deposit formation and allows conclusions to be drawn about the performance of chemical deposit control programs.

In the case of an organic fouling or organic biofilm as deposit 22, the deposit 22 forms on the inside surface of the wall 22 of the fluid vessel 20 and exhibits, e.g., a fur-like structure, that is, it contains fiber-like filaments that extend from the inside surface of the wall 22 of the fluid vessel 20 toward the interior of the fluid vessel 20.

In Figures 4a, 4b and 4c, schematic sectional views of a fluid vessel wall 21 in the region of a reflection area 12 with further kinds of a deposit 22 are shown. These examples of deposits 22 relate especially to the formation of inorganic deposits 22 such as CaCO₃., CaSo₄, calcium phosphate and/or iron oxide. Such inorganic deposits 22 tend to be generated in a disconnected (non-continuous) manner as shown in Figure 4a with spots of deposit having a lateral dimension in the range of 1 micrometer to 100 micrometer, particularly in the range of 5 micrometer to 50 micrometer, more particularly in the range of 8 micrometer to 12 micrometer, and a height dimension in the range of 1 micrometer to 100 micrometer, particularly in the range of 2 micrometer to 20 micrometer, more particularly in the range of 5 micrometer to 10 micrometer. In a further step of the generation of especially inorganic deposits 22, the spots of deposit 22 grow in height and the number of spots per area increase, as shown in Figure 4b. Furthermore, particles present in the fluid 25 are incorporated into the deposit layer 22, as represented schematically in Figure 4c by circles and/or ovals. According to the present invention, the generation of these different kinds of deposits 22 or stages in the generation of the deposit 22 can be differentiated by means of monitoring the evolution of the reflection signal 71, 72, 73, 74. These terms are further explained in connection with Figures 5 to 8.

As shown only in Figure 1, the device 10 preferably comprises a control unit 15, more preferably located inside a housing (not represented by a reference numeral) common to the device 10. The control unit 15 preferably comprises an evaluation module (not represented by a reference numeral). The evaluation module contains a conventional type of memory, in which comparative data can be stored. In addition, a comparator unit is available in which a measuring signal from the control unit 15 is present on the input side and which communicates with the memory via a signal line for the transmission of the comparative data. By means of the comparator unit, it is possible to compare the measuring signal with the comparative data in the memory. The result of the comparison can be used to control an input unit for a biocide or another deposit control agent so that the addition of expensive substances posing a health hazard can be optimized.

In Figures 5 to 8, a schematical time-domain diagram showing the emission signal 61 and first, second, third and fourth reflection signals 71, 72, 73, 74 respectively are shown. In all of these figures 5 to 8, an amplitude (reference sign "A", in arbitrary units) is schematically shown in the time-domain (i.e. versus time "t").

In Figures 5 and 6, the measuring situations of a deposit 22 are very schematically shown where the reflection area 12 comprises a material having an acoustic impedance corresponding to the second acoustic impedance (i.e. substantially different from the first acoustic impedance of the fluid 25). The situation represented in Figure 5 corresponds, e.g., to the case where virtually no deposit 22 is present on the reflection area 12. The relatively strong first reflection signal 71 corresponds to a comparably high degree of reflection and to a relatively low degree of scattering (effects of the ultrasonic wave at the fluid/deposit interface 51). The situation represented in Figure 6 corresponds to the case where there is a deposit 22 present on the reflection area 12 and the surface of that deposit (i.e. the surface of the deposit 22 with the fluid 25) is such that - integrated over the reflection area 12 struck by the ultrasonic emission signal 61 - there is a comparably low degree of reflection (or a reflection combined with a comparably high degree of scattering which means that the incident ultrasonic radiation is diverted in different directions). This can be due, .e.g., to a craggy surface of the deposit 22 as this can often be seen when inorganic scale deposits begin to grow. The relatively weak second reflection signal 72 corresponds to a comparably low degree of reflection and to a relatively high degree of scattering effects of the ultrasonic wave at the fluid/deposit interface 51. It is, of course, evident that the time required in order to change the situation corresponding to Figure 5 to the situation corresponding to Figure 6 depends on the fluid bearing system and on the process parameters that are more or less favorable the growth of the deposit 22.

In Figures 7 and 8, the measuring situations of a deposit 22 are very schematically shown where the reflection area 12 comprises a material having an acoustic impedance corresponding to the third acoustic impedance (i.e. substantially equal to the first acoustic impedance of the fluid 25). The situation represented in Figure 7 corresponds, e.g., to the case where virtually no deposit 22 is present on the reflection area 12. The relatively weak third reflection signal 73 corresponds to a comparably low degree of reflection and to a relatively high degree of scattering effects of the ultrasonic wave at the fluid/deposit interface 51. The situation represented in Figure 8 corresponds to the case where there is a deposit 22 present on the reflection area 12 and the difference in the acoustic impedance of the deposit 22 relative to the first acoustic impedance is superior to the difference between the first and third acoustic impedances such that there is a comparably high degree of reflection. This can be due, .e.g., to a biofilm or fouling deposit present on a polyurethane or polypropylene or polymeric or plastic surface (having the property of an acoustic impedance corresponding to the third acoustic impedance). The relatively strong fourth reflection signal 74 corresponds to a comparably high degree of reflection and to a relatively low degree of scattering effects of the ultrasonic wave at the fluid/deposit interface 51. It is, of course, evident that the time required in order to change the situation corresponding to Figure 7 to the situation corresponding to Figure 8 depends on the fluid bearing system and on the process parameters that are more or less favorable the growth of the deposit 22.

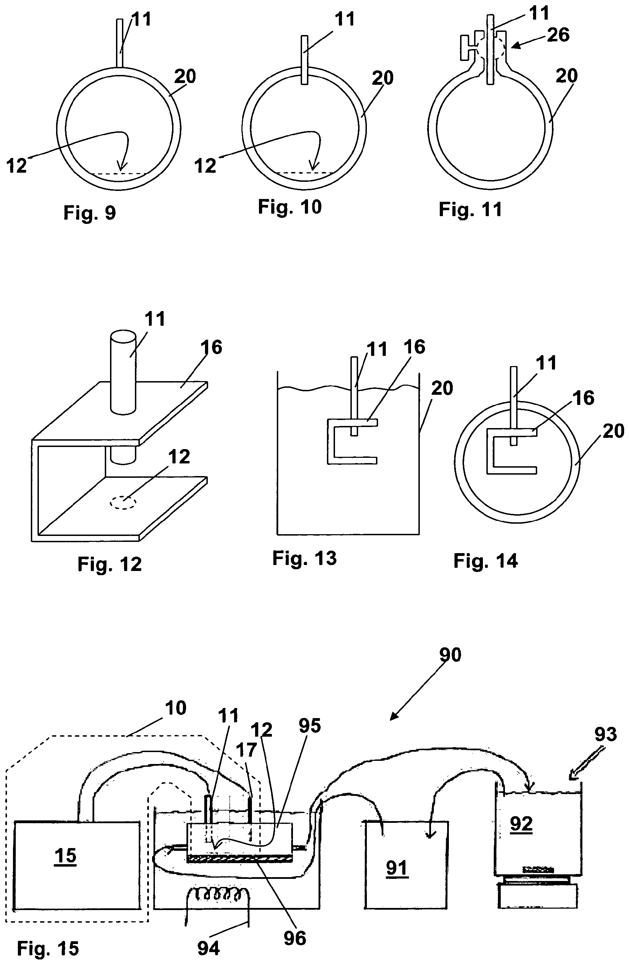

In Figures 9 to 14, schematic sectional or perspective views of different examples for holding a transducer relative to the fluid vessel are shown. In Figure 9, an embodiment of the ultrasonic transducer 11 mounted to the outside of the fluid vessel 20 is shown. This means that the ultrasonic transducer 11 is not directly in contact with the fluid 25 inside the fluid vessel 20. The reflection area 12 is schematically shown by means of a dashed line. Depending on the curvature of the wall of the fluid vessel 20 in the region of the reflection area 12, an insert or the like having a reduced curvature can be provided in the region of the reflection area 12. In Figure 10, an embodiment of the ultrasonic transducer 11 mounted to the inside of the fluid vessel 20 is shown. This means that ultrasonic transducer 11 is located such that at least part of the so-called head of the ultrasonic transducer 11, i.e. a (vibrating) active surface of the ultrasonic transducer 11, is provided in contact with the fluid 25 inside the fluid vessel 20. In the embodiment shown in Figure 10, the ultrasonic transducer 11 can, e.g., be mounted to the fluid vessel 20 by means of a screw thread in the wall of the fluid vessel 20. In Figure 11, an embodiment of the ultrasonic transducer 11 mounted to the inside of the fluid vessel 20 is shown. In this embodiment shown in Figure 11, the ultrasonic transducer 11 can be mounted or installed to the fluid vessel 20 via a valve 26 mounted in a wall of the fluid vessel 20. Different types of valves are possible to use in accordance with this embodiment, but ball valves or spherical valves are preferred. In Figure 12, an example of a holding means 16 is shown together with the ultrasonic transducer 11 and the reflection area 12 located on the holding means 16. The holding means 16 can, e.g., be provided as a U-shaped profile and the ultrasonic transducer 11 can be mounted relative to the holding means 16 with, e.g., two screw nuts. In Figures 13 and 14, the attachment of the ultrasonic transducer 11 by means of the holding means 16 is schematically shown for two situations, namely relative to a fluid vessel 20 in the form of a reservoir or tank (Figure 13) and relative to a fluid vessel 20 in the form of a tube (Figure 14)

### EXAMPLES

The device 10 according to the present invention was used in an experimental setup with an aqueous medium as the fluid 25. In Figure 15, a schematic sectional view of such an experimental setup structure 90 is shown. A measuring cell 95 comprises the ultrasonic transducer 11 and a reflection plate 96 of the measuring cell 95 providing the reflection area 12 opposite of the ultrasonic transducer 11. In the experimental setup structure 90, the reflection plate 96 is provided exchangeable. The deposit 22 is generated on the reflection plate 96. The ultrasonic transducer 11 is linked to a control unit 15 to form the inventive device 10. In the experimental setup structure 90, the device 10 further comprises a temperature sensor 17. The measuring cell 95 is maintained at a given first temperature by means of a heating means 94. A pump 91 is pumping the fluid 25 through the measuring cell 95 and towards a reservoir 92 which is maintained at given second temperature. Air is blown (indicated with an arrow 93 in Figure 15) to the surface of the fluid 25 in the reservoir 92 in order to enhance evaporation. The measuring cell 95, the reservoir 92 and the pump 91 are connected via flexible tubes.

### First example:

The device 10 was used within the experimental setup structure 90 with tap water of Krefeld, Germany, (approximately 2,5 Liters). The reflection plate 96 was a stainless steel plate of 3 Millimeters of thickness. The water in the reservoir 92 was maintained at the second temperature of 66° centigrade (by means of a heater and magnetic stirrer, not shown). The measuring cell 95 was maintained at a first temperature of 96° centigrade. The water is continuously pumped through the measuring cell 95 by means of the pump 91. By means of the evaporation (accelerated by the action of the air flow indicated at 93 in Figure 15) of the water in the reservoir 92, the fluid is continuously thickened. The evaporated water was regularly replaced by temperated tap water, but no elutriation occurred. After 216 minutes, the fluid 25 was entirely replaced by fresh temperated tap water, the pump 91 being only stopped for changing the flexible tubes and the measuring cell 95 was shortly vented.

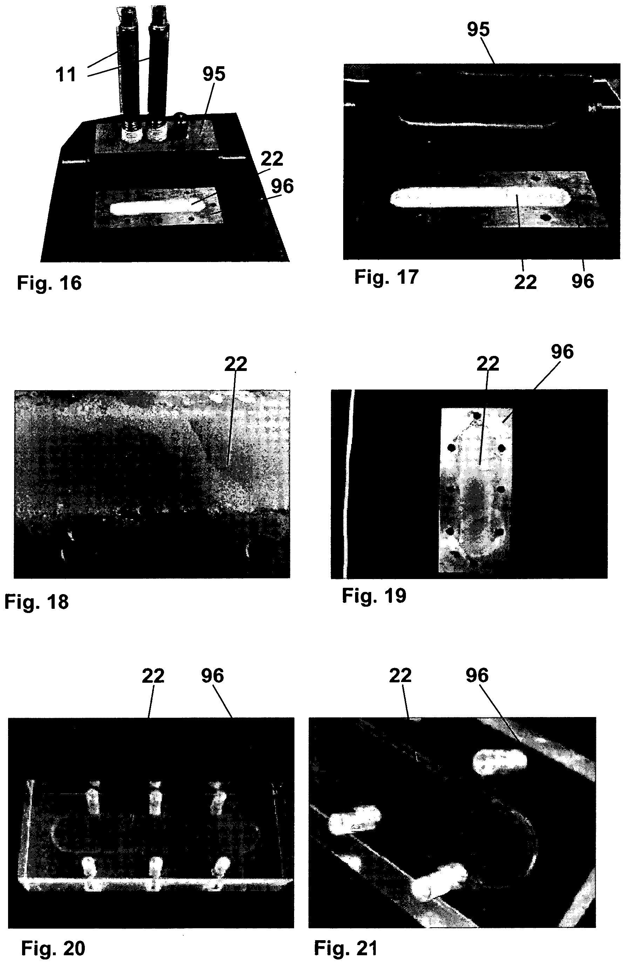

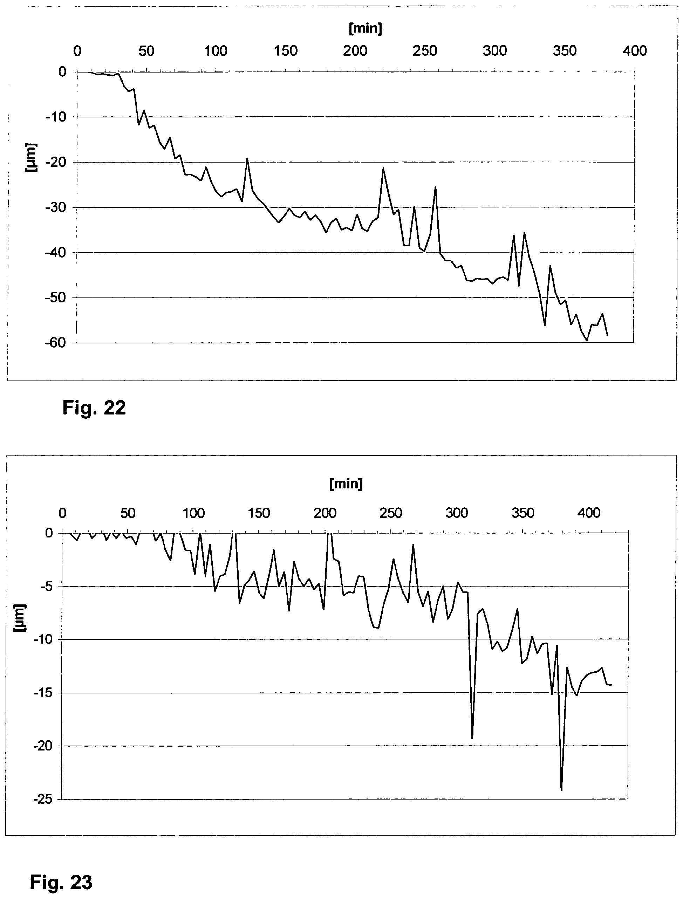

The results of the first example are depicted in Figures 16, 17 and 22. Figure 16 shows the measuring cell 95 (with two ultrasonic transducers 11 mounted to the measuring cell) together with the reflection plate 96 (with the deposit 22) after having finished the first example. Figure 17 shows the opened measuring cell 95 together with the reflection plate 96 (with the deposit 22) after having finished the first example. Figure 22 shows the variation (during a time of roughly 400 minutes (abscissa) of conducting the first example) of the measured distance (corresponding to the side scale of the ordinate showing a distance range between 0 micrometers and -60 micrometers) between the ultrasonic transducer 11 and the reflection area 12 on the reflection plate 96. It is clearly visible that the distance between the ultrasonic transducer 11 and the reflection area 12 is decreasing during the first 216 minutes and then again after the replacement of the fluid 25 (at 216 minutes) a further decrease of the distance between the ultrasonic transducer 11 and the reflection area 12 is clearly detectable.

### Second example:

The device 10 was used within the experimental setup structure 90 with tap water of Krefeld, Germany, (approximately 2,5 Liters). The reflection plate 96 was a stainless steel plate of 3 Millimeters of thickness. The water temperatures in the reservoir 92 and in the measuring cell 95 were identical to the first example. An elutriation of approximately 150 milliliter to 250 milliliter was performed every 30 minutes by means of fresh temperate water. In the second example, no complete replacement of the fluid 25 was performed.

The results of the second example are depicted in Figures 18, 19 and 23. Figures 18 and 19 show the reflection plate 96 (with the deposit 22) after having finished the second example. Figure 23 shows the variation (during a time of roughly 400 minutes (abscissa) of conducting the second example) of the measured distance (corresponding to the side scale of the ordinate showing a distance range between 0 micrometers and -25 micrometers) between the ultrasonic transducer 11 and the reflection area 12 on the reflection plate 96. It is clearly visible that due to the different conditions inside the measuring cell 95 during the second example (compared to the first example), the thickness of the deposit 22 layer is reduced because the variation of the measured distance is in the range of approximately 10 to 15 micrometers (during the time interval between approximately 70 minutes and 400 minutes) compared to a variation of the measured distance in the range of approximately 25 to 35 micrometers (during the time interval between approximately 60 minutes and 390 minutes) in the first example.

### Third example:

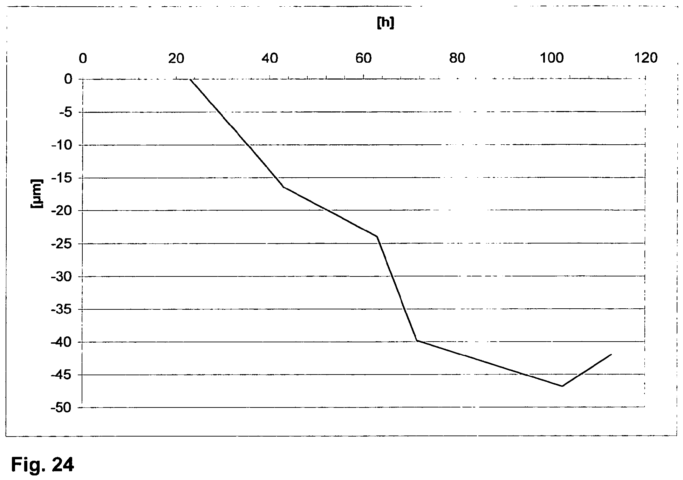

The device 10 was used within the experimental setup structure 90 with a fluid 25 containing 1,0 liter of tap water of Krefeld, Germany, 1,0 liter of surface water and 100g of Saccharose. The pH-value was at 7. The reflection plate 96 was an acrylic glass plate having an insert for providing a material in the reflection area 12 having an acoustic impedance (third acoustic impedance) comparable to water. The water was not heated (first temperature and second temperature at 26° centigrade).
The results of the second example are depicted in Figures 20, 21 and 24. Figures 20 and 21 show the reflection plate 96 (with the deposit 22) after having finished the third example. Figure 24 shows the variation (during a time of roughly 120 hours (abscissa) of conducting the third example) of the measured distance (corresponding to the ordinate showing a distance range between 0 micrometers and -50 micrometers) between the ultrasonic transducer 11 and the reflection area 12 on the reflection plate 96. It is clearly visible that the growing of a biofilm can be detected.

### List of reference numerals:

10 device
11 ultrasonic transducer
11' first ultrasonic transducer
11" second ultrasonic transducer
12 reflection area
13 first part of the reflection area
14 second part of the reflection area
15 control unit
16 holding means
17 temperature sensor
20 fluid vessel
21 wall of the fluid vessel
22 deposit
25 fluid
26 valve of the fluid vessel
28 outer wall surface
50 distance between the ultrasonic transducer and a fluid/deposit or fluid/wall interface
51 fluid/deposit interface
58 distance between the ultrasonic transducer and an outer wall surface
61 ultrasonic emission signal
71 first reflection signal
72 second reflection signal
73 third reflection signal
74 fourth reflection signal
90 experimental setup structure
91 pump
92 reservoir
93 air flow
94 heating means
95 measuring cell
96 reflection plate

## Claims

1. Method for a high precision measurement of a characteristic of a fouling and/or scaling deposit (22) inside a fluid vessel (20) or of a characteristic of a portion of the wall (21) inside a fluid vessel (20),
wherein an ultrasonic transducer (11) is used,
wherein a reflection area (12) is provided in a portion of the wall (21) or attached to a portion of the wall (21) of the fluid vessel (20) at a location substantially opposite of the ultrasonic transducer (11),
wherein the method comprises the steps of:
a) emitting an ultrasonic emission signal (61) by means of the ultrasonic transducer (11) and
b) measuring the distance (50) between the ultrasonic transducer (11) on the one hand and a fluid/deposit interface (51) or a fluid/wall interface (51) on the other hand in an absolute distance measurement by means of evaluating the time-domain reflective signal of the fluid/deposit or fluid/wall interface (51), wherein the fluid/deposit or fluid/wall interface (51) is either the interface of the fluid (25) with the deposit (22) on the reflection area (12) or the interface of the fluid (25) with the wall (21) in the reflection area (12),
wherein the time-domain resolution power is 1 ns or less than 1 ns,
wherein the temperature of the fluid is measured, wherein a temperature sensor (17) is used, wherein the distance between the ultrasonic transducer (11) on the one hand and a fluid/deposit interface (51) or a fluid/wall interface (51) on the other hand is derived from the arrival time of the reflective signal and the speed of sound of the fluid, the speed of sound being dependent on the temperature of the fluid, and wherein the ultrasonic transducer (11) is provided in contact with the fluid (25) or in contact with the inside of the fluid vessel (20).

2. Method according to claim 1, wherein in addition to measuring the time-domain reflective signal of the fluid/deposit or fluid/wall interface (51), the change of the acoustic impedance at the fluid/deposit or fluid/wall interface (51) is measured.

3. Method according to any of the preceding claims, wherein a characteristic of the fouling and/or scaling deposit (22) on the reflection area (12) or a characteristic of the wall (21) in the reflection area (12) is derived by means of the determination of the evolution of the change of the acoustic impedance at the fluid/deposit or fluid/wall interface (51).

4. Device (10) for a high precision measurement of a characteristic of a fouling and/or scaling deposit (22) inside a fluid vessel (20) or of a characteristic of a portion of the wall (21) inside a fluid vessel (20),
wherein the device (10) comprises an ultrasonic transducer (11),
wherein the device (10) further comprises a reflection area (12) in a portion of the wall (21) or attached to a portion of the wall (21) of the fluid vessel (20) at a location substantially opposite of the ultrasonic transducer (11),
wherein the distance (50) between the ultrasonic transducer (11) on the one hand and a fluid/deposit interface (51) or a fluid/wall interface (51) on the other hand is measured in an absolute distance measurement by means of evaluating the time-domain reflective signal of the fluid/deposit or fluid/wall interface (51),
wherein the fluid/deposit or fluid/wall interface (51) is either the interface of the fluid (25) with the deposit (22) on the reflection area (12) or the interface of the fluid (25) with the wall (21) in the reflection area (12),
wherein the time-domain resolution power of the device (10) is 1 ns or less than 1 ns, wherein the distance between the ultrasonic transducer (11) on the one hand and a fluid/deposit interface (51) or a fluid/wall interface (51) on the other hand is derived from the arrival time of the reflective signal and the speed of sound of the fluid, the speed of sound being dependent on the temperature of the fluid, wherein the ultrasonic transducer (11) is provided in contact with the fluid (25) or in contact with the inside of the fluid vessel (20), wherein the device (10) further comprises a temperature sensor (17).

5. Device (10) according to claim 4, wherein the temperature sensor (17) is located in an area adjacent to or integrated with the ultrasonic transducer (11).

6. Device (10) according to any of claims 4 or 5, wherein the fluid (25) has a first acoustic impedance for ultrasonic waves in the range from 1400 Ns/m³ to 1800 Ns/m³ and the reflection area (12) has at least partly a second acoustic impedance for ultrasonic waves, wherein
a) the second acoustic impedance exceeds the first acoustic impedance by at least 50%, preferably by at least 100%, more preferably by at least 200%, still more preferably by at least 500% and most preferably by at least 900%,
or wherein
b) the first acoustic impedance exceeds the second acoustic impedance by at least 40%, more preferably by at least 70%, still more preferably by at least 100% and most preferably by at least 150%.

7. Device (10) according to any of claims 4 to 6, wherein a characteristic of the fouling and/or scaling deposit (22) on the reflection area (12) or a characteristic of the wall (21) in the reflection area (12) is derived by means of a determination of the evolution of the change of the acoustic impedance at the fluid/deposit or fluid/wall interface (51).

8. Device (10) according to any of claims 4 to 7 wherein the fluid (25) has a first acoustic impedance for ultrasonic waves in the range from 1400 Ns/m³ to 1800 Ns/m³ and the reflection area (12) has at least partly a third acoustic impedance for ultrasonic waves, wherein
a) the third acoustic impedance exceeds the first acoustic impedance by 5% to 50%, preferably by 8% to 30%, more preferably by 10% to 20%,
or wherein
b) the first acoustic impedance exceeds the third acoustic impedance by 5% to 50%, preferably by 8% to 30%, more preferably by 10% to 20%.

9. Device (10) according to any of claims 6 or 7 wherein the material in the reflection area (12) having at least partly the third acoustic impedance is a polyurethane or polypropylene material or another polymeric or plastic material.

10. Device (10) according to any of the claims 4 to 8, wherein the reflection area (12) comprises a first part (13) and a second part (14), wherein the acoustic impedance of the first part (13) of the reflection area (12) corresponds to the second acoustic impedance, and wherein the acoustic impedance of the second part (14) of the reflection area (12) corresponds to the third acoustic impedance.

11. Device (10) according to claim 10, wherein the device (10) comprises first ultrasonic transducer (11') and a second ultrasonic transducer (11 "), the first ultrasonic transducer (11') being located substantially opposite of the first part (13) of the reflection area (12) and the second ultrasonic transducer (11") being located substantially opposite of the second part (14) of the reflection area (12).

12. Method for monitoring a characteristic of a fouling and/or scaling deposit (22) inside a fluid vessel (20) or of a characteristic of a portion of the wall (21) inside a fluid vessel (20) of a fluid bearing system, the method comprising the step of measuring the thickness of the deposit (22) or of the wall (21) by means of a device according to any of claims 4 to 11.

13. Method according to claim 12, the fluid bearing system comprising a regulating unit for a deposit control agent, the method comprising the steps of:
a) measuring the thickness of the deposit (22) with the device (10) to generate a measuring signal depending on the accumulation of the deposit (22) inside the fluid vessel (20); and
b) adjusting the supply of a deposit control agent by means of the regulating unit dependent on a comparison made between the measured thickness of the deposit (22) and a threshold value of the thickness of the deposit (22),
wherein the threshold value is preferably either predefined threshold value or a calculated threshold value.

## Patentansprüche

1. Verfahren für eine hochpräzise Messung von einer Eigenschaft von einer Verschmutzungs- und/oder Kalkablagerung (22) innerhalb eines Fluidbehälters (20) oder von einer Eigenschaft eines Teils einer Wand (21) innerhalb eines Fluidbehälters (20),
wobei ein Ultraschallwandler (11) verwendet wird,
wobei ein Reflexionsbereich (12) in einem Abschnitt der Wand (21) vorgesehen ist, oder
an einem Teil der Wand (21) des Fluidbehälters (20) an einer, im Wesentlichen dem Ultraschallwandler (11) gegenüberliegenden Stelle angebracht ist,
wobei das Verfahren die Schritte umfasst:
a) Emittieren eines Ultraschall-Emissionssignals (61) mittels des Ultraschallwandlers (11) und
b) Messen des Abstandes (50) zwischen dem Ultraschallwandler (11) einerseits und einer Fluid/Ablagerungs- Grenzfläche (51) oder einer Fluid/Wand-Grenzfläche (51) andererseits, in einer absoluten Abstandsmessung mittels Auswertung des Zeitbereichsreflexionssignals der Fluid/Ablagerung- oder Fluid/Wand-Grenzfläche (51),
wobei die Fluid/Ablagerungs- oder Fluid/Wand-Grenzfläche (51) entweder die Grenzfläche des Fluids (25) mit der Ablagerung (22) auf dem Reflexionsbereich (12), oder die Grenzfläche des Fluids (25) mit der Wand (21) in dem Reflexionsbereich (12) ist,
wobei die Zeitbereichsauflösungsleistung 1 ns oder weniger als 1 ns beträgt;
wobei die Temperatur des Fluids gemessen wird, wobei ein Temperatursensor (17) verwendet wird,
wobei der Abstand zwischen dem Ultraschallwandler (11) einerseits und eine Fluid/Ablagerungs-Grenzfläche (51) oder eine Fluid/Wand- Grenzfläche (51) andererseits von der Ankunftszeit des reflektierten Signals und der Schallgeschwindigkeit der Flüssigkeit abgeleitet wird, wobei die Schallgeschwindigkeit von der Temperatur des Fluids abhängt, und wobei der Ultraschallwandler (11) in Kontakt mit der Flüssigkeit (25) oder in Kontakt mit der Innenseite des Fluidbehälters (20) vorgesehen ist.

2. Verfahren nach Anspruch 1, wobei zusätzlich zu der Messung des Zeitbereichsreflexionssignals der Fluid/Ablagerungs- Fluid/Wand-Grenzfläche (51), die Änderung der akustischen Impedanz bei der Fluid/Ablagerungs- oder Fluid/Wand-Grenzfläche (51) gemessen wird.

3. Verfahren nach irgendeinem der vorhergehenden Ansprüche, wobei eine Eigenschaft der Verschmutzung und/oder Kalkablagerung (22) auf dem Reflexionsbereich (12) oder eine Eigenschaft der Wand (21) in dem Reflexionsbereich (12) abgeleitet wird mittels der Bestimmung der Entwicklung der Änderung der akustischen Impedanz an der Fluid/Ablagerungs- oder Fluid/Wand-Grenzfläche (51).

4. Vorrichtung (10) für eine hochpräzise Messung von einer Eigenschaft einer Verschmutzung- und/oder Kalkablagerung (22) innerhalb eines Fluidbehälters (20) oder einer Eigenschaft eines Teils der Wand (21) in einem Fluidbehälter (20),
wobei die Vorrichtung (10) ferner einen Ultraschallwandler (11) umfasst,
wobei die Vorrichtung (10) ferner einen Reflexionsbereich (12) in einem Abschnitt der Wand (21) oder an einem Abschnitt der Wand (21) des Fluidbehälters (20) an einer Stelle, die im Wesentlichen dem Ultraschallwandler (11) gegenüberliegt, aufweist
wobei der Abstand (50) zwischen dem Ultraschallwandler (11) einerseits und einer Fluid/Ablagerungs-Grenzfläche (51) oder einer Fluid/Wand-Grenzfläche (51) andererseits, in einer absoluten Abstandsmessung gemessen wird, indem das Zeitbereichsreflexionssignal der Fluid/Ablagerungs- oder Fluid/Wand-Grenzfläche (51) ausgewertet wird,
wobei die Fluid/Ablagerungs- oder die Fluid/Wand-Grenzfläche (51) entweder die Grenzfläche des Fluids (25) mit der Ablagerung (22) auf dem Reflexionsbereich (12) oder die Grenzfläche des Fluids (25) mit der Wand (21) in dem Reflexionsbereich (12) ist, wobei die Zeitbereichsauflösungsleistung der Vorrichtung (10) 1 ns oder weniger als 1 ns beträgt,
wobei der Abstand zwischen dem Ultraschallwandler (11) einerseits, und einer Fluid/Ablagerungs-Grenzfläche (51) oder einer Fluid/Wand-Grenzfläche (51) andererseits von der Ankunftszeit des reflektierten Signals und der Schallgeschwindigkeit des Fluids abgeleitet wird, die Schallgeschwindigkeit von der Temperatur des Fluids abhängt, wobei der Ultraschallwandler (11) in Kontakt mit dem Fluid (25) oder in Kontakt mit dem Inneren des Fluidgefäßes (20) vorgesehen ist, wobei das Gerät (10) ferner einen Temperatursensor (17) aufweist.

5. Vorrichtung (10) nach Anspruch 4, wobei der Temperatursensor (17) in einem an den Ultraschallwandler (11) angrenzenden Bereich angeordnet ist, oder in den Ultraschallwandler integriert ist.

6. Vorrichtung (10) nach irgendeinem der Ansprüche 4 oder 5, wobei das Fluid (25) eine erste akustische Impedanz für Ultraschallwellen im Bereich von 1400 Ns/m³ bis 1800 Ns/m³ hat und der Reflexionsbereich (12) zumindest teilweise eine zweite akustische Impedanz für Ultraschallwellen hat, wobei
a) die zweite akustische Impedanz die erste akustische Impedanz um mindestens 50% überschreitet, vorzugsweise um mindestens 100%, weiter vorzugsweise um mindestens 200%, noch weiter vorzugsweise um mindestens 500%, und am meisten vorzugsweise um mindestens 900%, überschreitet oder wobei
b) die erste akustische Impedanz die zweite akustische Impedanz um mindestens 40% überschreitet, weiter vorzugsweise um mindestens 70%, noch weiter vorzugsweise um mindestens 100%, und am meisten vorzugsweise um mindestens 150% überschreitet.

7. Vorrichtung (10) nach einem der Ansprüche 4 bis 6, wobei eine Eigenschaft der Verschmutzung und/oder Kalkablagerung (22) auf dem Reflexionsbereich (12) oder eine Eigenschaft der Wand (21) im Reflexionsbereich (12) von einer Bestimmung der Entwicklung der Änderung der akustischen Impedanz an der Fluid/Ablagerungs- oder Fluid/Wand-Grenzfläche (51) abgeleitet wird.

8. Vorrichtung (10) nach irgendeinem der Ansprüche 4 bis 7, wobei das Fluid (25) eine erste akustische Impedanz für Ultraschallwellen im Bereich von 1400 Ns/m³ bis 1800 Ns/m³ aufweist und der Reflexionsbereich (12) mindestens teilweise eine dritte akustische Impedanz für Ultraschallwellen aufweist, wobei
a) die dritte akustische Impedanz die erste akustische Impedanz um 5% bis 50% überschreitet, vorzugsweise um 8% bis 30%, weiter vorzugsweise um 10% bis 20% überschreitet, oder wobei
b) die erste akustische Impedanz die dritte akustische Impedanz um 5% bis 50% überschreitet, vorzugsweise 8% bis 30%, weiter vorzugsweise 10% bis 20% überschreitet.

9. Vorrichtung (10) nach irgendeinem der Ansprüche 6 oder 7, wobei das Material in dem Reflexionsbereich (12), das zumindest teilweise die dritte akustische Impedanz hat, ein Polyurethan oder Polypropylenmaterial oder ein anderes Polymer- oder Kunststoffmaterial ist.

10. Vorrichtung nach irgendeinem der Ansprüche 4 bis 8, wobei der Reflexionsbereich (12) einen ersten Teil (13) und einen zweiten Teil (14) umfasst, wobei die akustische Impedanz des ersten Teils (13) des Reflexionsbereichs (12) der zweiten akustischen Impedanz entspricht, und wobei die akustische Impedanz des zweiten Teils (14) der Reflexionsfläche (12) der dritten akustischen Impedanz entspricht.

11. Vorrichtung (10) nach Anspruch 10, wobei die Vorrichtung (10) einen ersten Ultraschallwandler (11') und einen zweiten Ultraschallwandler (11") umfasst, wobei der erste Ultraschallwandler (11') im Wesentlichen gegenüber dem ersten Teil (13) des Reflexionsbereichs (12) angeordnet ist, und der zweite Ultraschallwandler (11") im Wesentlichen gegenüber dem zweiten Teil (14) des Reflexionsbereichs (12) angeordnet ist.

12. Verfahren zum Überwachen einer Eigenschaft von einer Verschmutzungs- und/oder Kalkablagerung (22) innerhalb eines Fluidbehälters (20) oder von einer Eigenschaft eines Teils der Wand (21) innerhalb eines Fluidbehälters (20) eines Fluidlagersystems, das Verfahren umfassend den Schritt des Messens der Dicke der Ablagerung (22) oder der Wand (21) mittels einer Vorrichtung nach irgendeinem der Ansprüche 4 bis 11.

13. Verfahren nach Anspruch 12, wobei das Fluidlagersystem eine Reguliereinheit für ein Ablagerungskontrollmittel umfasst, wobei das Verfahren die Schritte umfasst:
a) Messen der Dicke der Ablagerung (22) mit der Vorrichtung (10), um ein Messsignal abhängig von der Ansammlung der Ablagerung (22) innerhalb des Fluidbehälters (20) zu erzeugen; und
b) Einstellen der Zufuhr eines Ablagerungskontrollmittels mittels der Reguliereinheit abhängig von einem Vergleich, der zwischen der gemessenen Dicke der Ablagerung (22) und einem Schwellenwert der Dicke der Ablagerung (22) durchgeführt wird, wobei der Schwellenwert vorzugsweise entweder ein vordefinierter Schwellenwert oder ein berechneter Schwellenwert ist.

## Revendications

1. Procédé de mesure de haute précision d'une caractéristique de dépôt de salissure et/ou de tartre (22) à l'intérieur d'un récipient de fluide (20), ou d'une caractéristique d'une partie de la paroi (21) à l'intérieur d'un récipient de fluide (20),
dans lequel un transducteur ultrasonique est utilisé (11),
dans lequel une zone de réflexion (12) est prévuée sur une partie de la paroi (21) ou est attachée à une partie de la paroi (21) du récipient de fluide (20) à un emplacement sensiblement en regard du transducteur ultrasonique (11),
dans lequel le procédé comprend les étapes consistant à :
a) émettre un signal d'émission ultrasonore (61) au moyen du transducteur ultrasonique (11), et
b) mesurer la distance (50) entre le transducteur ultrasonique (11) d'une part, et une interface fluide-dépôt (51) ou une interface fluide-paroi (51) d'autre part, dans une mesure de distance absolue, au moyen de l'évaluation du signal de réflexion dans le domaine temporel de l'interface fluide-dépôt ou fluide-paroi (51), dans lequel l'interface fluide-dépôt ou fluide-paroi (51) est soit l'interface du fluide (25) avec le dépôt (22) sur la zone de réflexion (12) soit l'interface du fluide (25) avec la paroi (21) dans la zone de réflexion (12),
dans lequel le pouvoir de résolution dans le domaine temporel est égal à 1 ns ou inférieur à 1 ns,
dans lequel la température du fluide est mesurée,
dans lequel un capteur de température (17) est utilisé, dans lequel la distance entre le transducteur ultrasonique (11) d'une part, et une interface fluide-dépôt (51) ou une interface fluide-paroi (51) d'autre part, est calculée d'après le temps d'arrivée du signal de réflexion et la vitesse du son dans le fluide, la vitesse du son étant fonction de la température du fluide, et
dans lequel le transducteur ultrasonique (11) est placé au contact du fluide (25) ou au contact de l'intérieur du récipient de fluide (20).

2. Procédé selon la revendication 1, dans lequel outre la mesure du signal de réflexion dans le domaine temporel de l'interface fluide-dépôt ou fluide-paroi (51), la variation de l'impédance acoustique à l'interface fluide-dépôt ou fluide-paroi (51) est mesurée.

3. Procédé selon l'une quelconque des revendications précédentes, dans lequel une caractéristique du dépôt de salissure et/ou de tartre (22) sur la zone de réflexion (12) ou une caractéristique de la paroi (21) dans la zone de réflexion (12) est calculée au moyen de la détermination de l'évolution de la variation de l'impédance acoustique à l'interface fluide-dépôt ou fluide-paroi (51).

4. Dispositif (10) de mesure de haute précision d'une caractéristique de dépôt de salissure et/ou de tartre (22) à l'intérieur d'un récipient de fluide (20) ou d'une caractéristique d'une partie de la paroi (21) à l'intérieur d'un récipient de fluide (20),
dans lequel le dispositif (10) comprend un transducteur ultrasonique (11),
dans lequel le dispositif (10) comprend en outre une zone de réflexion (12) sur une partie de la paroi (21) ou attachée à une partie de la paroi (21) du récipient de fluide (20), à un emplacement sensiblement en regard du transducteur ultrasonique (11),
dans lequel la distance (50) entre le transducteur ultrasonique (11) d'une part, et une interface fluide-dépôt (51) ou une interface fluide-paroi (51) d'autre part, est mesurée dans une mesure de distance absolue, au moyen de l'évaluation du signal de réflexion dans le domaine temporel de l'interface fluide-dépôt ou fluide-paroi (51),
dans lequel l'interface fluide-dépôt ou fluide-paroi (51) est soit l'interface du fluide (25) avec le dépôt (22) sur la zone de réflexion (12) soit l'interface du fluide (25) avec la paroi (21) dans la zone de réflexion (12),
dans lequel le pouvoir de résolution du dispositif (10) dans le domaine temporel est égal à 1 ns ou inférieur à 1 ns,
dans lequel la distance entre le transducteur ultrasonique (11) d'une part, et une interface fluide-dépôt (51) ou une interface fluide-paroi (51) d'autre part, est calculée d'après le temps d'arrivée du signal de réflexion et la vitesse du son dans le fluide, la vitesse du son étant fonction de la température du fluide,
dans lequel le transducteur ultrasonique (11) est placé au contact du fluide (25) ou au contact de l'intérieur du récipient de fluide (20),
dans lequel le dispositif (10) comprend en outre un capteur de température (17).

5. Dispositif (10) selon la revendication 4, dans lequel le capteur de température (17) est placé de préférence dans une zone adjacente ou intégrée au transducteur ultrasonique (11).

6. Dispositif (10) selon l'une quelconque des revendications 4 et 5, dans lequel le fluide (25) possède une première impédance acoustique pour les ondes ultrasonores, comprise dans la plage de 1400 Ns/m3 à 1800 Ns/m3 et la zone de réflexion (12) possède au moins en partie une deuxième impédance acoustique pour les ondes ultrasonores, dans lequel :
a) la deuxième impédance acoustique dépasse la première impédance acoustique d'au moins 50 %, de préférence d'au moins 100 %, de préférence encore d'au moins 200 %, mieux encore d'au moins 500 % et idéalement d'au moins 900 %, ou dans lequel :
b) la première impédance acoustique dépasse la deuxième impédance acoustique d'au moins 40 %, de préférence d'au moins 70 %, de préférence encore d'au moins 100 % et idéalement d'au moins 150 %.

7. Dispositif (10) selon l'une quelconque des revendications 4 à 6, dans lequel une caractéristique du dépôt de salissure et/ou de tartre (22) sur la zone de réflexion (12) ou une caractéristique de la paroi (21) dans la zone de réflexion (12) est calculée au moyen d'une détermination de l'évolution de la variation de l'impédance acoustique à l'interface fluide-dépôt ou fluide-paroi (51).

8. Dispositif (10) selon l'une quelconque des revendications 4 à 7, dans lequel le fluide (25) possède une première impédance acoustique pour les ondes ultrasonores, comprise dans la plage de 1400 Ns/m3 à 1800 Ns/m3 et la zone de réflexion (12) possède au moins en partie une troisième impédance acoustique pour les ondes ultrasonores, dans lequel :
a) la troisième impédance acoustique dépasse la première impédance acoustique de 5 % à 50 %, de préférence de 8 % à 30 %, de préférence encore de 10 % à 20 %,
ou dans lequel :
b) la première impédance acoustique dépasse la troisième impédance acoustique de 5 % à 50 %, de préférence de 8 % à 30 %, de préférence encore de 10 % à 20 %.

9. Dispositif (10) selon l'une quelconque des revendications 6 et 7, dans lequel le matériau de la zone de réflexion (12) possédant au moins en partie la troisième impédance acoustique est un matériau polyuréthane ou polypropylène ou une autre matière polymère ou plastique.

10. Dispositif (10) selon l'une quelconque des revendications 4 à 8, dans lequel la zone de réflexion (12) comprend une première partie (13) et une seconde partie (14), dans lequel l'impédance acoustique de la première partie (13) de la zone de réflexion (12) correspond à la deuxième impédance acoustique, et dans lequel l'impédance acoustique de la seconde partie (14) de la zone de réflexion (12) correspond à la troisième impédance acoustique.

11. Dispositif (10) selon la revendication 10, dans lequel le dispositif (10) comprend un premier transducteur ultrasonique (11') et un second transducteur ultrasonique (11"), le premier transducteur ultrasonique (11') étant placé sensiblement en regard de la première partie (13) de la zone de réflexion (12) et le second transducteur ultrasonique (11") étant placé sensiblement en regard de la seconde partie (14) de la zone de réflexion (12).

12. Procédé de surveillance d'une caractéristique de dépôt de salissure et/ou de tartre (22) à l'intérieur d'un récipient de fluide (20), ou d'une caractéristique d'une partie de la paroi (21) à l'intérieur d'un récipient de fluide (20) d'un système contenant un fluide, le procédé comprenant l'étape consistant à mesurer l'épaisseur du dépôt (22) ou de la paroi (21) au moyen d'un dispositif selon l'une quelconque des revendications 4 à 11.

13. Procédé selon la revendication 12, le système contenant un fluide comprenant une unité de régulation destinée à un agent anti-dépôt, le procédé comprenant les étapes consistant à :
a) mesurer l'épaisseur du dépôt (22) avec le dispositif (10) pour produire un signal de mesure en fonction de l'accumulation du dépôt (22) à l'intérieur du récipient de fluide (20) ; et
b) ajuster l'apport d'un agent anti-dépôt au moyen de l'unité de régulation, en fonction d'une comparaison effectuée entre l'épaisseur mesurée du dépôt (22) et une valeur de seuil de l'épaisseur du dépôt (22),
dans lequel la valeur de seuil est de préférence soit une valeur de seuil prédéfinie soit une valeur de seuil calculée.
